# EUROPEAN PATENT SPECIFICATION

(11) **EP 2 152 879 B1**
(45) Date of publication and mention of the grant of the patent: **14.11.2012**
(21) Application number: 08749943.0
(22) Date of filing: 30.04.2008
(51) Int. Cl.: C12N 15/113, A61K 31/7088

(54) **RNA ANTAGONIST COMPOUNDS FOR THE MODULATION OF BETA-CATENIN**
RNA-ANTAGONISTENVERBINDUNGEN ZUR MODULATION VON BETA-CATENIN
COMPOSÉS ANTAGONISTES DE L'ARN POUR LA MODULATION DE LA BÉTA CATÉNINE

(30) Priority: 01.05.2007 US 915371 P; 24.01.2008 US 23244
(43) Date of publication of application: 17.02.2010
(73) Proprietor: Santaris Pharma A/S, 2970 Hørsholm (DK)
(72) Inventor: WORM, Jesper, DK-1700 Copenhagen V (DK)
(74) Representative: Wroe, Stephanie
(86) International application number: PCT/EP2008/055365
(87) International publication number: WO 2008/132234

(56) References cited:
- WO-A-01/00872
- WO-A2-03/031937
- WO-A2-2004/045543
- US-A1- 2003 064 384
- US-A1- 2005 136 395
- US-A1- 2007 009 899
- VEERAMACHANEMI N.K. ET AL.: "Down-regulation of beta catenin inhibits the growth of esophageal carcinoma cells" THE JOURNAL OF THORACIC AND CARDIOVASCULAR SURGERY, vol. 127, January 2004 (2004-01), pages 92-98, XP002512177
- ROH H. ET AL.: "Suppression of beta-Catenin inhibits the neoplastic growth of APC-mutant colon cancer cells" CANCER RESEARCH, vol. 61, 1 September 2001 (2001-09-01), pages 6563-6568, XP002512178
- CANTER R.J. ET AL.: "Suppression of beta-Catenin by antisense oligomers augments tumor response to isolated limb perfusion in a rodent model of adenomatous polyposis coli-mutant colon cancer" ANNALS OF SURGICAL ONCOLOGY, SPRINGER-VERLAG, NE, vol. 12, no. 9, 1 September 2005 (2005-09-01), pages 733-742, XP019369730 ISSN: 1534-4681
- GREEN D.W. ET AL.: "Beta-Catenin antisense treatment decreases beta-Catenin expression andn tumor growth rate in colon carcinoma xenografts" JOURNAL OF SURGICAL RESEARCH, vol. 101, 2001, pages 16-20, XP002512179
- EMANUELE S. ET AL.: "Sodium Butyrate induces apoptosis in human epatoma cells by a mitochondria/caspase pathway, associated with degradation of beta-catenin, pRb and Bcl-XL" EUROPEAN JOURNAL OF CANCER, vol. 40, 2004, pages 1441-1452, XP002512180
- LI M. ET AL.: "Antitumor activity and chemosensitization effects of novel antisense oligonucleotides targeting beta-catenin" PROCEEDINGS OF THE AMERICAN ASSOCIATION FOR CANCER RESEARCH ANNUAL MEETING, vol. 46, April 2005 (2005-04), page 137, XP001537058
- HEASMAN J. ET AL.: "Overexpression of Cadherins and underexpression of beta-Catenin inhibit dorsal mesoderm induction in early Xenopus embryos" CELL, CELL PRESS, CAMBRIDGE, NA, US, vol. 79, no. 5, 2 December 1994 (1994-12-02), pages 791-803, XP024244579 ISSN: 0092-8674 [retrieved on 1994-12-02]
- KIM K. ET AL.: "Tissue-specific expression of beta-Catenin in normal mesenchyme and uveal melanomas and its effect on invasiveness" EXPERIMENTAL CELL RESEARCH, ACADEMIC PRESS, US, vol. 245, 1 January 1998 (1998-01-01), pages 79-90, XP002930314 ISSN: 0014-4827

## Description

### FIELD OF THE INVENTION

The invention relates to oligomeric compounds (oligomers), which target beta-catenin mRNA in a cell, leading to reduced expression of beta-catenin. Reduction of beta-catenin expression is beneficial for a range of medical disorders, such as hyperproliferative diseases, including cancer. The invention provides therapeutic compositions comprising oligomers and methods for modulating the expression of beta-catenin using said oligomers, including methods of treatment.

### BACKGROUND

Beta-catenin (also known as cadherin-associated protein and β-Catenin) is a member of the catenin family of cytosolic proteins and a pivotal player in the signalling pathway initiated by Wnt proteins, mediators of several developmental processes. Beta-catenin undergoes phosphorylation upon growth factor stimulation resulting in reduced cell adhesion.

The role of beta-catenin in the development of cancer has been shown to be regulated by the expression product of the APC (adenomatous polyposis of the colon) gene. The APC tumor suppressor protein binds to beta-catenin, while beta-catenin was shown to interact with Tcf and Lef transcription factors. Morin *et al.* report that APC protein down-regulates the transcriptional activation mediated by beta-catenin and Tcf-4 in colon cancer. Their results indicate that the regulation of beta-catenin is critical to APC's tumor suppressive effect and that this regulation can be circumvented by mutations in either APC or beta-catenin. Several studies report on the detection of mutations in beta-catenin in various cancer cell lines and abnormally high amounts of beta-catenin have been found in melanoma cell lines.

Morin *et al.* showed that mutations of beta-catenin which affect phosphorylation sites rendered the cells insensitive to APC-mediated down-regulation of beta-catenin and that this disrupted mechanism was critical to colorectal tumorigenesis (Morin et al., Science, 1997, 275, 1787-1790).

U.S. Patent No. 6,066,500 to Bennett et al. describes antisense compounds, compositions and methods for modulating the expression of beta-catenin.

WO01/00872 discloses antisense compounds for modulating the expression of Beta catenin,

Veeramachanemi et al (J Thorac and Cardiovas Surg, 2004, 127: 92-8) suggest that down-regulation of beta catenin would inhibit the growth of human oesophageal cancer.

Roh et al (Cancer Research, 2001, 61: 6563-6568) disclose the use of antisense oligonucleotides to down-regulate β-catenin expression in APC-mutant human colon carcinoma cells.

Canter et al (Annals of Surgical Oncology, 2005, 12(9): 733-742) disclose the use of antisense oligodeoxynucleotides to determine if they result in downregulation of β-catenin and whether this improves tumour response to isolated limb perfusion in a heterotropic model of human CRC.

Green et al (J of Surgical Research, 2001, 101: 16-20) report that the treatment of *APC*-mutant colorectal carcinoma xenografts with β-catenin antisense resulted in a dose-dependent down-regulation in β-catenin protein expression as shown by western blotting.

Emanuele et al (European J of Cancer, 2004, 40: 1441-1452) disclose that in HuH-6 cells pre-treatment with β-catenin antisense ODN reduced the content of β-catenin and anticipated the onset of apoptosis at 8h of exposure to butyrate.

Li et al (Proceedings of the American Association for Cancer Research, 2005, 46: 137) disclose a study in which to develop antisense oligonucelotides against β-catenin as anti-cancer agents.

WO03/031937 discloses identifying polymorphic markers for herbicide resistance in weeds.

WO04/045543 discloses sequence specific gene silencing through the use of siRNA technology.

Considering the indication of beta-catenin in the development of cancer, there remains a need for agents capable of effectively inhibiting beta-catenin function.

### SUMMARY OF THE INVENTION

The invention provides an oligomer of between 10-50 nucleobases in length which comprises a contiguous nucleobase sequence of a total of between 10-50 nucleobases, wherein said contiguous nucleobase sequence is at least 80% homologous to a corresponding region of a nucleic acid which encodes a mammalian beta-catenin.

The invention provides oligomer of between 10-50 nucleobases in length which comprises a contiguous nucleobase sequence of a total of between 10-50 nucleobases, wherein said contiguous nucleobase sequence is at least 80% homologous to a corresponding region of a nucleic acid sequence selected from the group consisting of SEQ ID NO 173 and SEQ ID NOs 174 - 193.

The invention provides oligomers of between 10-50 nucleobases in length which comprises a contiguous nucleobase sequence of a total of between 10-50 nucleobases, wherein said contiguous nucleobase sequence is at least 80% homologous to a corresponding region of a nucleic acid sequence selected from the group consisting of SEQ ID No 1 - 132.

The invention further provides a conjugate comprising the oligomer according to the invention, such as a conjugate which, in addition to the nucleobase sequence of the oligomer comprises at least one non-nucleotide (i.e non-nucleobase) or non-polynucleotide moiety covalently attached to the oligomer of the invention. The non-nucleotide or non-polynucleotide moiety may consist of or comprise a sterol group such as cholesterol, or other moiety that facilitates entry into the cell.

The invention provides for an conjugate comprising the oligomer according to the invention, such as a conjugate which, in addition to the nucleobase sequence of the oligomer comprises a polymeric conjugate containing positively charged moieties, such as polyethylene glycol (PEG) - *i.e.* the oligomer according to the invention may, optionally, be pegylated.

The invention provides for pharmaceutical composition comprising the oligomer or as defined conjugate of the invention, and a pharmaceutically acceptable diluent, carrier, salt or adjuvant.

The invention further provides for an oligomer according to the invention, for use in medicine.

The invention further provides for the use of the oligomer of the invention for the manufacture of a medicament for the treatment of one or more of the diseases referred to herein, such as a disease selected from: hyperproliferative diseases such as those referred to herein, such as cancer.

The invention further provides for an oligomer according to the invention, for use for the treatment of one or more of the diseases referred to herein, such as cancer.

Pharmaceutical and other compositions comprising the oligomer or conjugate of the invention are also provided. Further provided are methods of down-regulating the expression of beta-catenin in cells or tissues comprising contacting said cells or tissues, *in vitro* or *in vivo,* with one or more of the oligomers, conjugates or compositions of the invention.

Also disclosed are methods of treating an animal or a human, suspected of having or being prone to a disease or condition, associated with expression, or over-expression, of beta-catenin, by administering to said animal or human a therapeutically or prophylactically effective amount of one or more of the oligomers, conjugates or compositions of the invention.

Further, methods of using oligomers for the inhibition of expression of beta-catenin, and for treatment of diseases associated with activity of beta-catenin are provided.

The invention provides for a method for treating an a disease or disorder, such as those referred to herein, such as a hyperproliferative disease such as cancer, said method comprising administering an oligomer, a conjugate, or a pharmaceutical composition according to the invention to a patient in need thereof.

The invention provides for a method of inhibiting or reducing the expression of beta-catenin in a cell or a tissue, the method comprising the step of contacting said cell or tissue with an oligomer, a conjugate, or a pharmaceutical composition according to the invention so that expression of beta-catenin is inhibited or reduced.

The invention provides for a method of triggering apoptosis in a cell, such as a cancer cell, said method comprising the step of contacting said cell or tissue with an oligomer, a conjugate, or a pharmaceutical composition according to the invention so that either expression of beta-catenin is inhibited or reduced and/or apoptosis is triggered.

The invention further provides for an oligomer which comprises or consists of a contiguous nucleobase sequence which is at least 80% homologous to a region corresponding to a sequence selected from the group consisting of SEQ ID NOs 1-132 and SEQ ID NOs 174-193, such as a sequence selected from the group consisting of SEQ ID NO 189, 192, 58 and 103.

The invention further provides for an oligomer which comprises or consists of a contiguous nucleobase sequence which corresponds to an equivalent sequence present in a sequence selected from SEQ ID NO 174-193, such as SEQ ID NO 189 or 192.

The invention further provides for an oligomer which comprise or consist of a sequence selected from the group consisting of SEQ ID NO 133 - 174, such as SEQ ID NO 168 or 171, or which comprise or consist of a contiguous nucleobase sequence which corresponds to said sequence.

### RELATED CASES

The following related applications are hereby mentioned : US 60/915,371 and US 61/023,244.

### BRIEF DESCRIPTION OF THE FIGURES

**Figure 1****:** The beta-catenin target sequences that correspond to the oligonucleotide sequences of SEQ ID NOs: 1, 16, 17, 18, 33, 34, 49, 50, 51, 52, 53, 54, 55, 56, 57, 58, 73, 88, 103 and 118, respectively, are shown in bold and underlined, indicating their position in the beta-catenin transcript (Genbank Accession number NM_001904 - SEQ ID NO: 173).
**Figure 2****:** Beta-catenin expression normalized to GAPDH 24 hours after transfection of SW480 cells.
**Figure 3****:** Beta-catenin protein content in SW480 cells transfected with oligonucleotides at 1 and 5 nM concentration measured by Western blot. Tubulin staining was used as loading control and SEQ ID NO: 194 was used as a scrambled control oligonucleotide.
**Figure 4****:** Cellular proliferation measured using MTS assay in HCT116 cells transfected with oligonucleotides at 25 nM concentration measured as OD490 at different timepoints after transfection. Mock transfected cells and cells transfected with SEQ ID NO: 194 (scrambled control) were used as controls.
**Figure 5****:** Beta-catenin mRNA expression relative to saline treated control in mouse liver measured by QPCR after treatment with 3 x 25 mg/kg oligonucleotide i.v. Beta-catenin expression was normalized to GAPDH and results were plotted relative to saline treated control.

### DETAILED DESCRIPTION OF THE INVENTION

### The Oligomer

The present invention employs oligomeric compounds (referred herein as oligomers), for use in modulating the function of nucleic acid molecules encoding mammalian beta-catenin, such as the beta-catenin nucleic acid shown in SEQ ID NO 173, and naturally occurring allelic variants of such nucleic acid molecules encoding mammalian beta-catenin.

The term "oligomer" in the context of the present invention, refers to a molecule formed by covalent linkage of two or more nucleobases (*i.e*. an oligonucleotide). Herein, each single nucleobases may also be referred to as a monomer or unit. The oligomer consists or comprises of a contiguous nucleobase sequence of between 10 - 50 nucleobases in length. An oligomer may comprise of natural nucleotide units, e.g. ribonucleic acid (**RNA**) or deoxyribonucleic acid (DNA), or nucleic acid analogues, or mixtures thereof as referred to herein, preferably including Locked Nucleic Acid (LNA). It thus includes oligomer composed of naturally occurring nucleobases, sugars and internucleobase linkages as well as oligomers having non-naturally-occurring nucleobases which function similarly or with specific improved functions. Fully or partly modified or substituted oligomers are often preferred over native forms because of several desirable properties of such oligomers, such as the ability to penetrate a cell membrane, good resistance to extra- and/or intracellular nucleases and high affinity and specificity for the nucleic acid target. LNA analogue is particularly preferred, for example, regarding the above-mentioned properties. Therefore, in a highly preferable embodiment an oligomer according to the invention is built up of both nucleotide and nucleotide analogue units, such as LNA units, or from different groupings of nucleotide analogue units, to form an oligonucleotide of between 10-50, preferably 10-25 nucleobases, more preferably 10-16 nucleobases, and even more preferably between 12-16 nucleobases.

In various embodiments, the oligomer of the invention does not comprise RNA (units). It is preferred that the compound according to the invention is a linear molecule or is synthesised as a linear molecule. The oligomer is a single stranded molecule, and preferably does not comprise short regions of, for example, at least 3, 4 or 5 contiguous nucleobases, which are complementary to equivalent regions within the same oligomer (*i.e*. duplexes) - in this regards, the oligomer is not (essentially) double stranded. In various embodiments, the oligomer is essentially not double stranded, such as is not a siRNA. The oligomer comprises or consists of a contiguous nucleobase sequence. In various embodiments, the oligomer of the invention may consist entirely of the contiguous nucleobase region. In various embodiments, the nucleobase sequence of the oligomer consists of the contiguous nucleobase sequence.

### The Target

The term "target nucleic acid", as used herein refers to the DNA encoding mammalian beta-catenin polypeptide, such as human beta-catenin, such as SEQ ID NO 173. beta-catenin encoding nucleic acids or naturally occurring variants thereof, and RNA nucleic acids derived therefrom, preferably mRNA, such as pre-mRNA, although preferably mature mRNA. In various embodiments, for example when used in research or diagnostics the "target nucleic acid" may be a cDNA or a synthetic oligonucleotide derived from the above DNA or RNA nucleic acid targets. The oligomeric compound according to the invention is preferably capable of hybridising to the target nucleic acid.

The term "target nucleic acid", may, in various embodiments, be e.g., the human gene exons having Accession Nos. X89579, X89593, X89592, X89591, X89588, X89585, X89584 and X89578), or a mammalian beta-catenin mRNA such as SEQ ID NO 173. Examples of other mammalian beta-catenin mRNAs include, but are not limited to, those having Accession Numbers NM_001098209, NM_0010987210 (human beta-catenin mRNA); NM_007614 (mouse beta-catenin mRNA); NM_053357 (rat beta-catenin mRNA); NM-001122762, DQ267491 (horse beta-catenin mRNA); NM_214367 (pig beta-catenin mRNA); BC119949, BT030683 (cow beta-catenin mRNA). It should, of course, be recognised that the above accession numbers refer to the cDNA sequence and not the mRNA sequence per se - the mature mRNA sequence can, of course be derived directly from the cDNA sequence - thymine (T) residues being replaced by uracil (U) residues.

As used herein, "hybridisation" means hydrogen bonding, which may be Watson-Crick, Hoogsteen, reversed Hoogsteen hydrogen bonding, etc. between complementary nucleotide/nucleobase bases. Watson and Crick showed approximately fifty years ago that deoxyribo nucleic acid (DNA) is composed of two strands which are held together in a helical configuration by hydrogen bonds formed between opposing complementary nucleobases in the two strands. The four nucleobases, commonly found in DNA are guanine (G), adenine (A), thymine (T) and cytosine (C) of which the G nucleobase pairs with C, and the A nucleobase pairs with T. In RNA the nucleobase thymine is replaced by the nucleobase uracil (U), which similarly to the T nucleobase pairs with A. The chemical groups in the nucleobases that participate in standard duplex formation constitute the Watson-Crick face. Hoogsteen showed a couple of years later that the purine nucleobases (G and A) in addition to their Watson-Crick face have a Hoogsteen face that can be recognised from the outside of a duplex, and used to bind pyrimidine oligonucleotides via hydrogen bonding, thereby forming a triple helix structure.

It is highly preferred that the compounds of the invention are capable of hybridizing to the target nucleic acid, such as the mRNA.

Suitably the oligomer or conjugate of the invention is capable of inhibiting such as down-regulating expression of the beta-catenin gene. In various embodiments, the oligomers of the invention bind to the target nucleic acid and effect inhibition of expression of at least 10% or 20% compared to the normal expression level, more preferably at least a 30%, 40%, 50%, 60%, 70%, 80%, 90% or 95% inhibition compared to the normal expression level. In some embodiments, such modulation is seen when using between 1 and 25nM of the oligomer or conjugate of the invention. In the same or a different embodiment, the inhibition of expression is less than 100%, such as less than 98% inhibition, less than 95% inhibition, less than 90% inhibition, less than 80% inhibition, such as less than 70% inhibition. Modulation of expression level may be determined by measuring protein levels, *e.g.* by the methods such as SDS-PAGE followed by western blotting using suitable antibodies raised against the target protein. Alternatively, modulation, such as inhibition, of expression levels can be determined by measuring levels of mRNA, *e.g.* by northern blotting or quantitative RT-PCR. When measuring via mRNA levels, the level of inhibition or down-regulation when using an appropriate dosage, such as between 1 and 25nM, is, in various embodiments, typically to a level of between 10-20% the normal levels in the absence of the compound of the invention.

The invention therefore provides a method of down-regulating or inhibiting the expression of beta-catenin protein and/or mRNA in a cell which is expressing beta-catenin protein and/or mRNA, said method comprising administering the oligomer or conjugate according to the invention to said cell to down-regulating or inhibiting the expression of beta-catenin protein and/or mRNA in said cell. Suitably the cell is a mammalian cell such as a human cell. The administration may occur, in various embodiments, *in vitro.* The administration may occur, in various embodiments, *in vivo.*

The term "target nucleic acid", as used herein refers to the DNA encoding mammalian beta-catenin polypeptide, such as human beta-catenin, such as SEQ ID NO: 173. Beta-catenin encoding nucleic acids or naturally occurring variants thereof, and RNA nucleic acids derived therefrom, preferably mRNA, such as pre-mRNA, although preferably mature mRNA. In various embodiments, for example when used in research or diagnostics the "target nucleic acid" may be a cDNA or a synthetic oligonucleotide derived from the above DNA or RNA nucleic acid targets. The oligomer according to the invention is preferably capable of hybridising to the target nucleic acid.

The term "naturally occurring variant thereof" refers to variants of the beta-catenin polypeptide or nucleic acid sequence which exist naturally within the defined taxonomic group, such as mammalian, such as mouse, monkey, and preferably human. Typically, when referring to "naturally occurring variants" of a polynucleotide the term also may encompass any allelic variant of the beta-catenin encoding genomic DNA which are found at the Chromosome Chr 3: 41.22 - 41.26 Mb by chromosomal translocation or duplication, and the RNA, such as mRNA derived therefrom. When referenced to a specific polypeptide sequence, e.g., the term also includes naturally occurring forms of the protein which may therefore be processed, e.g. by co- or post-translational modifications, such as signal peptide cleavage, proteolytic cleavage, glycosylation, etc.

The term "at least one" comprises the integers larger than or equal to 1, such as 1, 2, 3, 4, 5, 6, 7, 8, 9, 10, 11, 12, 13, 14, 15, 16, 17, 18, 19, 20 and so forth.

In various embodiments, such as when referring to the nucleic acid or protein targets of the compounds of the invention, the term "at least one" includes the terms "at least two" and at "least three" and "at least four", likewise the term "at least two" may comprise the terms at "least three" and "at least four".

### Sequences

The oligomers of the invention may target the human beta-catenin mRNA, and as such they comprise or consist of a contiguous nucleobase sequence which corresponds to a nucleotide sequence present in SEQ ID NO: 173, or a sequence selected from the group consisting of SEQ ID NOS: 1 - 132, wherein said oligomer (or contiguous nucleobase portion thereof) may optionally comprise one, two, or three mismatches against said selected sequence. In various embodiments, the oligonucleotide sequences consist or comprise a sequence of 10-16 (contiguous) nucleobases. Examples of oligonucleotide sequences comprising 16 nucleobases are shown in SEQ ID NOS: 1, 16, 17, 18, 33, 34, 49, 50, 51, 52, 53, 54, 55, 56, 57, 58, 73, 88, 103, and 118. Shorter sequences can be derived therefrom. Longer sequences may include all, or at least 10 nucleobases from the respective SEQ ID NO.

| **Table 1 Antisense oligonucleotide sequences (Motifs)** | | | |
|---|---|---|---|
| **SEQ ID NO** | **Sequence (5'-3')** | **Length (bases)** | **Description** |
| SEQ ID NO: 1 | GAAAGCTGATGGACCA | 16 | Antisense oligo sequence |
| SEQ ID NO: 2 | GAAAGCTGATGGACC | 15 | as above |
| SEQ ID NO: 3 | AAAGCTGATGGACCA | 15 | as above |
| SEQ ID NO: 4 | GAAAGCTGATGGAC | 14 | as above |
| SEQ ID NO: 5 | AAAGCTGATGGACC | 14 | as above |
| SEQ ID NO: 6 | AAGCTGATGGACCA | 14 | as above |
| SEQ ID NO: 7 | GAAAGCTGATGGA | 13 | as above |
| SEQ ID NO: 8 | AAAGCTGATGGAC | 13 | as above |
| SEQ ID NO: 9 | AAGCTGATGGACC | 13 | as above |
| SEQ ID NO: 10 | AGCTGATGGACCA | 13 | as above |
| SEQ ID NO: 11 | GAAAGCTGATGG | 12 | as above |
| SEQ ID NO: 12 | AAAGCTGATGGA | 12 | as above |
| SEQ ID NO: 13 | AAGCTGATGGAC | 12 | as above |
| SEQ ID NO: 14 | AGCTGATGGACC | 12 | as above |
| SEQ ID NO: 15 | GCTGATGGACCA | 12 | as above |
| SEQ ID NO: 16 | CAGACTTAAAGATGGC | 16 | as above |
| SEQ ID NO: 17 | CAGAATCCACTGGTGA | 16 | as above |
| SEQ ID NO: 18 | GCACTGCCATTTTAGC | 16 | as above |
| SEQ ID NO: 19 | GCACTGCCATTTTAG | 15 | as above |
| SEQ ID NO: 20 | CACTGCCATTTTAGC | 15 | as above |
| SEQ ID NO: 21 | GCACTGCCATTTTA | 14 | as above |
| SEQ ID NO: 22 | CACTGCCATTTTAG | 14 | as above |
| SEQ ID NO: 23 | ACTGCCATTTTAGC | 14 | as above |
| SEQ ID NO: 24 | GCACTGCCATTTT | 13 | as above |
| SEQ ID NO: 25 | CACTGCCATTTTA | 13 | as above |
| SEQ ID NO: 26 | ACTGCCATTTTAG | 13 | as above |
| SEQ ID NO: 27 | CTGCCATTTTAGC | 13 | as above |
| SEQ ID NO: 28 | GCACTGCCATTT | 12 | as above |
| SEQ ID NO: 29 | CACTGCCATTTT | 12 | as above |
| SEQ ID NO: 30 | ACTGCCATTTTA | 12 | as above |
| SEQ ID NO: 31 | CTGCCATTTTAG | 12 | as above |
| SEQ ID NO: 32 | TGCCATTTTAGC | 12 | as above |
| SEQ ID NO: 33 | GTAATAGCCAAGAATT | 16 | as above |
| SEQ ID NO: 34 | ACTCTGCTTGTGGTCC | 16 | as above |
| SEQ ID NO: 35 | ACTCTGCTTGTGGTC | 15 | as above |
| SEQ ID NO: 36 | CTCTGCTTGTGGTCC | 15 | as above |
| SEQ ID NO: 37 | ACTCTGCTTGTGGT | 14 | as above |
| SEQ ID NO: 38 | CTCTGCTTGTGGTC | 14 | as above |
| SEQ ID NO: 39 | TCTGCTTGTGGTCC | 14 | as above |
| SEQ ID NO: 40 | ACTCTGCTTGTGG | 13 | as above |
| SEQ ID NO: 41 | CTCTGCTTGTGGT | 13 | as above |
| SEQ ID NO: 42 | TCTGCTTGTGGTC | 13 | as above |
| SEQ ID NO: 43 | CTGCTTGTGGTCC | 13 | as above |
| SEQ ID NO: 44 | ACTCTGCTTGTG | 12 | as above |
| SEQ ID NO: 45 | CTCTGCTTGTGG | 12 | as above |
| SEQ ID NO: 46 | TCTGCTTGTGGT | 12 | as above |
| SEQ ID NO: 47 | CTGCTTGTGGTC | 12 | as above |
| SEQ ID NO: 48 | TGCTTGTGGTCC | 12 | as above |
| SEQ ID NO: 49 | CCACCAGCTTCTACAA | 16 | as above |
| SEQ ID NO: 50 | GAGTCCAAAGACAGTT | 16 | as above |
| SEQ ID NO: 51 | ACCCACTTGGCAGACC | 16 | as above |
| SEQ ID NO: 52 | GCACAAACAATGGAAT | 16 | as above |
| SEQ ID NO: 53 | GCAGCTACTCTTTGGA | 16 | as above |
| SEQ ID NO: 54 | CTCCCTCAGCTTCAAT | 16 | as above |
| SEQ ID NO: 55 | GCAGTCTCATTCCAAG | 16 | as above |
| SEQ ID NO: 56 | TATCCACCAGAGTGAA | 16 | as above |
| SEQ ID NO: 57 | CATCCATGAGGTCCTG | 16 | as above |
| SEQ ID NO: 58 | CCATCTTGTGATCCAT | 16 | as above |
| SEQ ID NO: 59 | CCATCTTGTGATCCA | 15 | as above |
| SEQ ID NO: 60 | CATCTTGTGATCCAT | 15 | as above |
| SEQ ID NO: 61 | CATCTTGTGATCC | 14 | as above |
| SEQ ID NO: 62 | CATCTTGTGATCCA | 14 | as above |
| SEQ ID NO: 63 | ATCTTGTGATCCAT | 14 | as above |
| SEQ ID NO: 64 | CCATCTTGTGATC | 13 | as above |
| SEQ ID NO: 65 | CATCTTGTGATCC | 13 | as above |
| SEQ ID NO: 66 | ATCTTGTGATCCA | 13 | as above |
| SEQ ID NO: 67 | TCTTGTGATCCAT | 13 | as above |
| SEQ ID NO: 68 | CCATCTTGTGAT | 12 | as above |
| SEQ ID NO: 69 | CATCTTGTGATC | 12 | as above |
| SEQ ID NO: 70 | ATCTTGTGATCC | 12 | as above |
| SEQ ID NO: 71 | TCTTGTGATCCA | 12 | as above |
| SEQ ID NO: 72 | CTTGTGATCCAT | 12 | as above |
| SEQ ID NO: 73 | AAGCAAGCAAAGTCAG | 16 | as above |
| SEQ ID NO: 74 | AAGCAAGCAAAGTCA | 15 | as above |
| SEQ ID NO: 75 | AGCAAGCAAAGTCAG | 15 | as above |
| SEQ ID NO: 76 | AAGCAAGCAAAGTC | 14 | as above |
| SEQ ID NO: 77 | AGCAAGCAAAGTC | 14 | as above |
| SEQ ID NO: 78 | GCAAGCAAAGTCAG | 14 | as above |
| SEQ ID NO: 79 | AAGCAAGCAAAGT | 13 | as above |
| SEQ ID NO: 80 | AGCAAGCAAAGTC | 13 | as above |
| SEQ ID NO: 81 | GCAAGCAAAGTCA | 13 | as above |
| SEQ ID NO: 82 | CAAGCAAAGTCAG | 13 | as above |
| SEQ ID NO: 83 | AAGCAAGCAAAG | 12 | as above |
| SEQ ID NO: 84 | AGCAAGCAAAGT | 12 | as above |
| SEQ ID NO: 85 | GCAAGCAAAGTC | 12 | as above |
| SEQ ID NO: 86 | CAAGCAAAGTCA | 12 | as above |
| SEQ ID NO: 87 | AAGCAAAGTCAG | 12 | as above |
| SEQ ID NO: 88 | GAAATTGCTGTAGCAG | 16 | as above |
| SEQ ID NO: 89 | GAAATTGCTGTAGCA | 15 | as above |
| SEQ ID NO: 90 | AAATTGCTGTAGCAG | 15 | as above |
| SEQ ID NO: 91 | GAAATTGCTGTAGC | 14 | as above |
| SEQ ID NO: 92 | AAATTGCTGTAGCA | 14 | as above |
| SEQ ID NO: 93 | AATTGCTGTAGCAG | 14 | as above |
| SEQ ID NO: 94 | GAAATTGCTGTAG | 13 | as above |
| SEQ ID NO: 95 | AAATTGCTGTAGC | 13 | as above |
| SEQ ID NO: 96 | AATTGCTGTAGCA | 13 | as above |
| SEQ ID NO: 97 | ATTGCTGTAGCAG | 13 | as above |
| SEQ ID NO: 98 | GAAATTGCTGTA | 12 | as above |
| SEQ ID NO: 99 | AAATTGCTGTAG | 12 | as above |
| SEQ ID NO: 100 | AATTGCTGTAGC | 12 | as above |
| SEQ ID NO: 101 | ATTGCTGTAGCA | 12 | as above |
| SEQ ID NO: 102 | TTGCTGTAGCAG | 12 | as above |
| SEQ ID NO: 103 | GTGTTCTACACCATTA | 16 | as above |
| SEQ ID NO: 104 | GTGTTCTACACCATT | 15 | as above |
| SEQ ID NO: 105 | TGTTCTACACCATTA | 15 | as above |
| SEQ ID NO: 106 | GTGTTCTACACCAT | 14 | as above |
| SEQ ID NO: 107 | TGTTCTACACCATT | 14 | as above |
| SEQ ID NO: 108 | GTTCTACACCATTA | 14 | as above |
| SEQ ID NO: 109 | GTGTTCTACACCA | 13 | as above |
| SEQ ID NO: 110 | TGTTCTACACCAT | 13 | as above |
| SEQ ID NO: 111 | GTTCTACACCATT | 13 | as above |
| SEQ ID NO: 112 | TTCTACACCATTA | 13 | as above |
| SEQ ID NO: 113 | GTGTTCTACACC | 12 | as above |
| SEQ ID NO: 114 | TGTTCTACACCA | 12 | as above |
| SEQ ID NO: 115 | GTTCTACACCAT | 12 | as above |
| SEQ ID NO: 116 | TTCTACACCATT | 12 | as above |
| SEQ ID NO: 117 | TCTACACCATTA | 12 | as above |
| SEQ ID NO: 118 | AACATGAAATAGATCC | 16 | as above |
| SEQ ID NO: 119 | AACATGAAATAGATC | 15 | as above |
| SEQ ID NO: 120 | ACATGAAATAGATCC | 15 | as above |
| SEQ ID NO: 121 | AACATGAAATAGAT | 14 | as above |
| SEQ ID NO: 122 | ACATGAAATAGATC | 14 | as above |
| SEQ ID NO: 123 | CATGAAATAGATCC | 14 | as above |
| SEQ ID NO: 124 | AACATGAAATAGA | 13 | as above |
| SEQ ID NO: 125 | ACATGAAATAGAT | 13 | as above |
| SEQ ID NO: 126 | CATGAAATAGATC | 13 | as above |
| SEQ ID NO: 127 | ATGAAATAGATCC | 13 | as above |
| SEQ ID NO: 128 | AACATGAAATAG | 12 | as above |
| SEQ ID NO: 129 | ACATGAAATAGA | 12 | as above |
| SEQ ID NO: 130 | CATGAAATAGAT | 12 | as above |
| SEQ ID NO: 131 | ATGAAATAGATC | 12 | as above |
| SEQ ID NO: 132 | TGAAATAGATCC | 12 | as above |

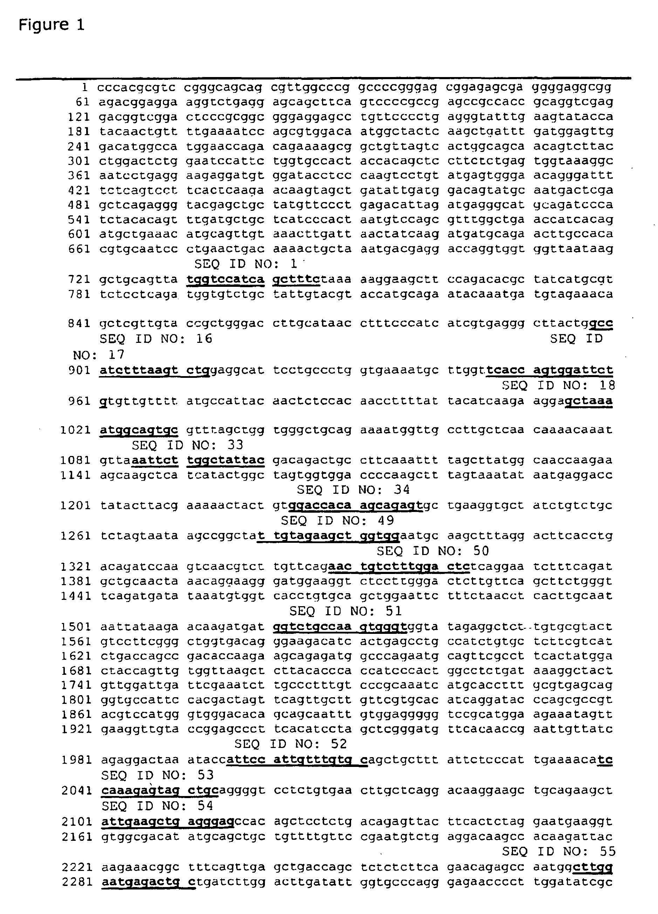

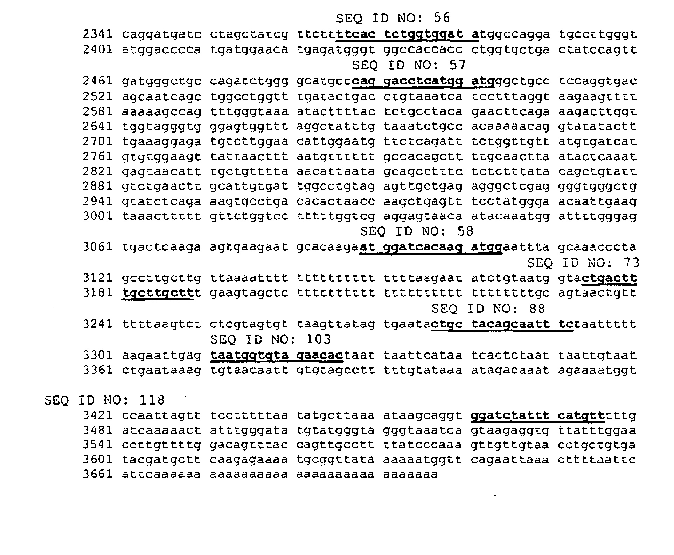

The invention further provides target sequences in the beta-catenin gene, in particular those corresponding to (i.e. the reverse complement of) SEQ ID NOs 1-132, wherein antisense oligonucleotides (oligomers) corresponding to said target sequences are capable of down-regulating beta-catenin. For example, target sequences which correspond to the antisense oligonucleotide sequences SEQ ID NOs: 1, 16, 17, 18, 33, 34, 49, 50, 51, 52, 53, 54, 55, 56, 57, 58, 73, 88, 103 and 118, respectively, are shown in Figure 1 (bold and underlined, with the corresponding oligo SEQ ID NOs indicated above).

The oligomer of the invention may, suitably, comprise or consist or be derived from (such as comprising a corresponding contiguous nucleobase sequence found within said SEQ ID), a sequence selected from SEQ ID NOS: 174 - 193. Preferably, the sequence comprises a sequence of 10-16 nucleobases. Examples of sequences comprising 16 nucleobases are shown in a SEQ ID selected from the group consisting of SEQ ID NOS: 1, 16, 17, 18, 33, 34, 49, 50, 51, 52, 53, 54, 55, 56, 57, 58, 73, 88, 103, and 118. Sequences which have fewer nucleobases, such as 10, 11, 12, 13, 14, or 15, may therefore have a contiguous nucleobase sequence which corresponds to a sub-sequence, for example of at least 8, at least 9, at least 10, at least 11, at least 12, at least 13, at least 14 or 15, contiguous nucleobases found in SEQ ID NOS: 1, 16, 17, 18, 33, 34, 49, 50, 51, 52, 53, 54, 55, 56, 57, 58, 73, 88, 103, and 118. Hence, shorter sequences can be derived therefrom. Longer sequences may include all, or at least 10 nucleobases from those exemplified SEQ ID NOs. Typically if only a portion of the sequence of the oligonucleotide is present in a SEQ ID selected from SEQ ID NOS: 1, 16, 17, 18, 33, 34, 49, 50, 51, 52, 53, 54, 55, 56, 57, 58, 73, 88, 103, and 118, then the remaining portion of the sequence of the oligonucleotide is homologous to the corresponding nucleotides flanking said SEQ ID. Two interesting sequence motifs are SEQ ID NO 58 and SEQ ID NO 103.

Specific designs of oligomers of the invention are also disclosed, for example those shown in SEQ ID NOS: 133-152, in particular SEQ ID NOs: 133, 134, 135, 136, 138, 141, 148, 149, 150, 151 and 152. Specific designs of LNA oligonucleotides are also disclosed, for example those shown in SEQ ID NOS: 153-172, in particular SEQ ID NOS: 153, 154, 155, 156, 158, 161, 168, 169, 170, 171 and 172. In preferred embodiments, the oligomers of the invention are considered to be potent inhibitors of beta-catenin mRNA and protein expression.

In certain embodiments, the oligomer may comprise or consist of a contiguous nucleobase sequence which is fully complementary (perfectly complementary) to the equivalent region of a nucleic acid which encodes a mammalian beta-catenin.

However, in some embodiments, the oligomer may include 1, 2, 3, or 4 (or more) mismatches, when hybridising to the target sequence and still sufficiently bind to the target to show the desired effect, i.e. down-regulation of the target. Mismatches may, for example, be compensated by increased length of the oligomer nucleobase sequence and/or an increased number of nucleotide analogues, such as LNA, present within the nucleobase sequence.

In various embodiments, the contiguous nucleobase sequence comprises no more than 3, such as no more than 2 mismatches to the target sequence, such as to the corresponding region of a nucleic acid which encodes a mammalian beta-catenin.

In various embodiments, the contiguous nucleobase sequence comprises no more than a single mismatch to the target sequence, such as the corresponding region of a nucleic acid which encodes a mammalian beta-catenin.

The nucleobase sequence of the oligomers of the invention or the contiguous nucleobase sequence is preferably at least 80% homologous to a corresponding sequence selected from the group consisting of SEQ ID NOS: 1 - 132, such as at least 85%, at least 90%, at least 91%, at least 92%, at least 93%, at least 94%, at least 95%, at least 96%, at least 97%, at least 98%, at least 99%, homologous, such as 100% homologous (identical).

The nucleobase sequence of the oligomers of the invention or the contiguous nucleobase sequence is preferably at least 80% homologous to a corresponding sequence present in SEQ ID NO: 173, such as at least 85%, at least 90%, at least 91%, at least 92%, at least 93%, at least 94%, at least 95%, at least 96%, at least 97%, at least 98%, at least 99%, homologous, such as 100% homologous (identical).

The nucleobase sequence of the oligomers of the invention or the contiguous nucleobase sequence is preferably at least 80% complementary to a sub-sequence present in SEQ ID NO: 173, such as at least 85%, at least 90%, at least 91%, at least 92%, at least 93%, at least 94%, at least 95%, at least 96% complementary, at least 97%, at least 98%, at least 99%, such as 100% complementary (perfectly complementary).

In various embodiments, the oligomer (or contiguous nucleobase portion thereof) is selected from, or comprises, one of the sequences selected from the group consisting of SEQ ID NOS: 1-132 or SEQ ID NOS: 174-193, or a sub-sequence of at least 10 contiguous nucleobases thereof, wherein said oligomer (or contiguous nucleobase portion thereof) may optionally comprise one, two, or three mismatches against said selected sequence.

In various embodiments the sub-sequence may consist of 11, 12, 13, 14, 15, 16, 17, 18, 19, 20, 21, 22, 23, 24, 25, 26, 27, 28, or 29 contiguous nucleobases, such as between 10 - 15, 12 - 25, 12 -22, such as between 12-18 nucleobases. Suitably, in various embodiments, the sub-sequence is of the same length as the contiguous nucleobase sequence of the oligomer of the invention.

However, it is recognised that, in various embodiments the nucleobase sequence of the oligomer may comprise additional 5' or 3' nucleobases, such as, independently, 1, 2, 3, 4 or 5 additional nucleobases 5' and/or 3', which are non-complementary to the target sequence. In this respect the oligomer of the invention, may, in various embodiments, comprise a contiguous nucleobase sequence which is flanked 5' and or 3' by additional nucleobases. In various embodiments, the 3' end of the contiguous nucleobase sequence is flanked by 1, 2 or 3 DNA or RNA units. 3' DNA units are frequently used during solid state synthesis of oligomers. In various embodiments, which may be the same or different, the 5' end of the contiguous nucleobase sequence is flanked by 1, 2 or 3 DNA or RNA units. In various embodiments the additional 5' or 3' nucleobases are naturally occurring nucleotides, such as DNA or RNA. In various embodiments, the additional 5' or 3' nucleobases may represent region D as referred to in the context of gapmer oligomers herein.

In various embodiments the oligomer according to the invention consists or comprises of a nucleobase sequence according to SEQ ID NO:1, or a sub-sequence of thereof.

In various embodiments the oligomer according to the invention consists or comprises of a nucleobase sequence according to SEQ ID NO: 16, or a sub-sequence of thereof.

In various embodiments the oligomer according to the invention consists or comprises of a nucleobase sequence according to SEQ ID NO: 17, or a sub-sequence of thereof.

In various embodiments the oligomer according to the invention consists or comprises of a nucleobase sequence according to SEQ ID NO:18, or a sub-sequence of thereof.

In various embodiments the oligomer according to the invention consists or comprises of a nucleobase sequence according to SEQ ID NO:33, or a sub-sequence of thereof.

In various embodiments the oligomer according to the invention consists or comprises of a nucleobase sequence according to SEQ ID NO:34, or a sub-sequence of thereof.

In various embodiments the oligomer according to the invention consists or comprises of a nucleobase sequence according to SEQ ID NO:49, or a sub-sequence of thereof.

In various embodiments the oligomer according to the invention consists or comprises of a nucleobase sequence according to SEQ ID NO:50, or a sub-sequence of thereof.

In various embodiments the oligomer according to the invention consists or comprises of a nucleobase sequence according to SEQ ID NO:51, or a sub-sequence of thereof.

In various embodiments the oligomer according to the invention consists or comprises of a nucleobase sequence according to SEQ ID NO:52, or a sub-sequence of thereof.

In various embodiments the oligomer according to the invention consists or comprises of a nucleobase sequence according to SEQ ID NO:53, or a sub-sequence of thereof.

In various embodiments the oligomer according to the invention consists or comprises of a nucleobase sequence according to SEQ ID NO:54, or a sub-sequence of thereof.

In various embodiments the oligomer according to the invention consists or comprises of a nucleobase sequence according to SEQ ID NO:55, or a sub-sequence of thereof.

In various embodiments the oligomer according to the invention consists or comprises of a nucleobase sequence according to SEQ ID NO:56, or a sub-sequence of thereof.

In various embodiments the oligomer according to the invention consists or comprises of a nucleobase sequence according to SEQ ID NO:57, or a sub-sequence of thereof.

In various embodiments the oligomer according to the invention consists or comprises of a nucleobase sequence according to SEQ ID NO:58, or a sub-sequence of thereof.

In various embodiments the oligomer according to the invention consists or comprises of a nucleobase sequence according to SEQ ID NO:73, or a sub-sequence of thereof.

In various embodiments the oligomer according to the invention consists or comprises of a nucleobase sequence according to SEQ ID NO:88, or a sub-sequence of thereof.

In various embodiments the oligomer according to the invention consists or comprises of a nucleobase sequence according to SEQ ID NO: 103, or a sub-sequence of thereof.

In various embodiments the oligomer according to the invention consists or comprises of a nucleobase sequence according to SEQ ID NO:118, or a sub-sequence of thereof.

When determining "homology" or "complementarity" between the oligomers of the invention (or contiguous nucleobase sequence) and the nucleic acid which encodes the mammalian beta-catenin, such as those disclosed herein, the determination of homology may be made by a simple alignment with the corresponding nucleobase sequence of the compound of the invention and the corresponding region of the nucleic acid which encodes the mammalian beta-catenin (or target nucleic acid), or complement thereof, and the homology is determined by counting the number of bases which align and dividing by the total number of contiguous nucleobases in the compound (oligomer) of the invention, and multiplying by 100. In such a comparison, if gaps exist, it is preferable that such gaps are merely mismatches rather than areas where the number of nucleobases within the gap differ between the nucleobase sequence of the invention and the target nucleic acid.

Amino acid and polynucleotide homology may be determined using ClustalW algorithm using standard settings: see http://www.ebi.ac.uk/emboss/align/index.html. Method: EMBOSS::water (local): Gap Open = 10.0, Gap extend = 0.5, using Blosum 62 (protein), or DNAfull for nucleotide/nucleobase sequences. Such alignments can also be used to identify regions of the nucleic acids encoding beta-catenin from human and a different mammalian species, such as monkey, mouse and/or rat, where there are sufficient stretches of nucleic acid complementarity to allow the design of oligonucleotides which target both the human beta-catenin target nucleic acid, and the corresponding nucleic acids present in the different mammalian species, such as oligomers which consist or comprise of regions of at least 10, such as at least 12, such as at least 14, such as at least 16, such as at least 18, such as 11, 12, 13, 14, 15, 16, 17 or 18 contiguous nucleobases which are 100% complementary to the equivalent regions of both the nucleic acid encoding beta-catenin from humans and the nucleic acid(s) encoding beta-catenin from the different mammalian species.

In various embodiments the oligomer of the invention comprises or consists of a contiguous nucleobase seqeunce which is at least 80%, such as at least 85%, such as at least 90%, such as at least 95%, such as at least 98%, such as at least 99%, or 100% homologous to the corresponding region of both the nucleic acid encoding beta-catenin from humans and a nucleic acid(s) encoding beta-catenin from the different mammalian species, such as the mouse nucleic acid encoding beta-catenin - (*i.e.* 80% - 100% complementary to both the equivalent region to both the human and mouse sequence). It is preferable that the contiguous nucleobase seqeunce of the oligomer is 100% complementary to the equivalent region of the human beta-catenin mRNA.

The terms "corresponding to" and "corresponds to" refer to the comparison between the nucleobase sequence of the oligomer or contiguous nucleobase sequence and the equivalent nucleotide sequence of i) the reverse complement of the nucleic acid target, such as the mRNA which encodes the beta-catenin protein, such as SEQ ID NO: 173, and/or ii) the sequence of nucleotides provided herein such as the group consisting of SEQ ID NOS: 1 - 132 or SEQ ID Nos 174-193. Nucleotide analogues are compared directly to their equivalent or corresponding nucleotides.

The terms "corresponding nucleotide analogue" and "corresponding nucleotide" are intended to indicate that the nucleobase in the nucleotide analogue and the naturally occurring nucleotide are identical. For example, when the 2-deoxyribose unit of the nucleotide is linked to an adenine, the "corresponding nucleotide analogue" contains a pentose unit (different from 2-deoxyribose) linked to an adenine.

### Length

The oligomer comprises or consists of a contiguous nucleobase sequence of a total of between 10 - 50 nucleobases, such as 10, 11, 12, 13, 14, 15, 16, 17, 18, 19, 20, 21, 22, 23, 24, 25, 26, 27, 28, 29 or 30 contiguous nucleobases in length.

In various embodiments, the oligomers comprise or consist of a contiguous nucleobase sequence of a total of between 10 - 25, 10 - 24, 12 - 25, 12 - 24, or 10 - 22, such as 12 - 18, such as 13 - 17 or 12 - 16, such as 13, 14, 15, 16 contiguous nucleobases in length.

In various embodiments, the oligomers comprise or consist of a contiguous nucleobase sequence of a total of 10, 11, 12, 13, or 14 contiguous nucleobases in length.

In various embodiments, the oligomer according to the invention consists of no more than 22 nucleobases, such as no more than 20 nucleobases, such as no more than 18 nucleobases, such as 15, 16 or 17 nucleobases. In various embodiments the oligomer of the invention comprises less than 20 nucleobases.

### Nucleotides and nucleotide analogues

The term "nucleotide" as used herein, refers to a glycoside comprising a sugar moiety, a base moiety and a covalently linked phosphate group and covers both naturally occurring nucleotides, such as DNA or RNA, preferably DNA. Non-naturally occurring nucleotides comprising modified sugar and/or base moieties are referred to as "nucleotide analogues" herein. The term "nucleobase" is used to encompass both natural (DNA- or RNA-type) nucleotides as well as nucleotide analogues.

Non-naturally occurring nucleotides include nucleotides which have modified sugar moieties, such as bicyclic nucleotides or 2' modified nucleotides, such as 2' substituted nucleotides.

"Nucleotide analogues" are variants of natural nucleotides, such as DNA or RNA nucleotides, by virtue of modifications in the sugar and/or base moieties. Analogues could in principle be merely "silent" or "equivalent" to the natural nucleotides in the context of the oligonucleotide, *i.e*. have no functional effect on the way the oligonucleotide works to inhibit target gene expression. Such "equivalent" analogues may nevertheless be useful if, for example, they are easier or cheaper to manufacture, or are more stable to storage or manufacturing conditions, or represent a tag or label. Preferably, however, the analogues will have a functional effect on the way in which the oligomer works to inhibit expression; for example by producing increased binding affinity to the target and/or increased resistance to intracellular nucleases and/or increased ease of transport into the cell.

The term "nucleobase" is used as a collective term which encompasses both nucleotides and nucleotide analogues. A nucleobase sequence is a sequence which comprises at least two nucleotides or nucleotide analogues. In various embodiments the nucleobase sequence may comprise of oniy nucleotides, such as DNA units, in an alternative embodiment, the nucleobase sequence may comprise of only nucleotide analogues, such as LNA units, or in various embodiments the nucleobase sequence may comprise both nucleotides and nucleotide analogue units.

The term "nucleic acid" is defined as a molecule formed by covalent linkage of two or more nucleotides.

The terms "nucleic acid" and "polynucleotide" are used interchangeable herein.

The "base moiety" of a nucleobase covers the base of both naturally occurring a well as non-naturally occurring nucleobases. Thus, the base moiety covers not only the known purine and pyrimidine heterocycles but also heterocyclic analogues and tautomeres thereof.

Examples of such base moieties include, but are not limited to adenine, guanine, cytosine, thymidine, uracil, xanthine, hypoxanthine, 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

In various embodiments, at least one of the nucleobases present in the oligomer comprises a modified base, such as is selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

Specific examples of nucleoside/nucleotide analogues are described by *e.g.* Freier & Altmann; Nucl. Acid Res., 1997, 25, 4429-4443 and Uhlmann; Curr. Opinion in Drug Development, 2000, 3(2), 293-213, and in Scheme 1:

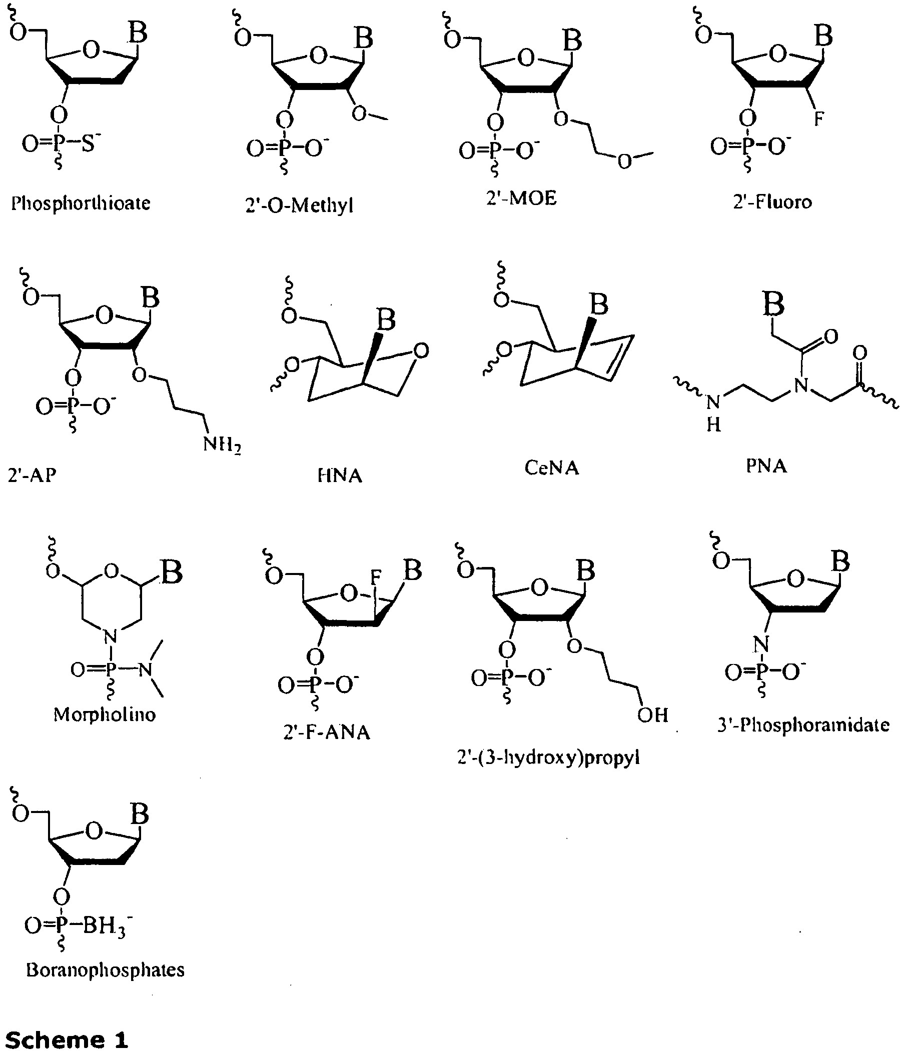

The oligomer may thus comprise or consist of a simple sequence of natural occurring nucleotides - preferably 2'-deoxynucleotides (referred to here generally as "DNA"), but also possibly ribonucleotides (referred to here generally as "RNA"), or a combination of such naturally occurring nucleotides and one or more non-naturally occurring nucleotides, *i.e*. nucleotide analogues. Such nucleotide analogues may suitably enhance the affinity of the oligomer for the target sequence.

Examples of suitable and preferred nucleotide analogues are described in WO2007/031091, or are referenced therein. One preferred modification of the nucleotide includes nucleotides where the sugar moiety is modified to provide a 2'-substituent group or to produce a bridged (locked nucleic acid - LNA) structure which enhances binding affinity and probably also provides some increased nuclease resistance; modifying the internucleotide linkage from its normal phosphodiester to one that is more resistant to nuclease attack, such as phosphorothioate or boranophosphate - these two, being cleavable by RNase H, also allow that route of antisense inhibition in modulating the beta-catenin expression. Incorporation of affinity-enhancing nucleotide analogues in the oligomer, such as LNA or 2'-substituted sugars, can allow the size of the specifically binding oligomer to be reduced, and may also reduce the upper limit to the size of the oligomer before non-specific or aberrant binding takes place.

In various embodiments the oligomer comprises at least 2 nucleotide analogues. In some embodiments, the oligomer comprises from 3-8 nucleotide analogues, *e.g*. 6 or 7 nucleotide analogues. In some preferred embodiments, at least one of said nucleotide analogues is a locked nucleic acid (LNA); for example at least 3 or at least 4, or at least 5, or at least 6, or at least 7, or 8, of the nucleotide analogues may be LNA. In some embodiments all the nucleotide analogues may be LNA.

It will be recognised that when referring to a preferred nucleotide sequence motif or nucleotide sequence, which consists of only nucleotides, the oligomers of the invention which are defined by that sequence may comprise a corresponding nucleotide analogue in place of one or more of the nucleotides present in said sequence, such as LNA units or other nucleotide analogues, which raise the duplex stability/Tₘ of the oligomer/target duplex (*i.e*. affinity enhancing nucleotide analogues).

In various embodiments, any mismatches between the nucleotide sequence of the oligomer and the target sequence are preferably found in regions outside the affinity enhancing nucleotide analogues (for example regions A and C), such as within region B as referred to herein, and/or region D as referred to herein, and/or at the site of non modified such as DNA nucleotides in the oligonucleotide, and/or in regions which are 5' or 3' to the contiguous nucieobase sequence.

Examples of such modification of the nucleotide include modifying the sugar moiety to provide a 2'-substituent group or to produce a bridged (locked nucleic acid) structure which enhances binding affinity and may also provide increased nuclease resistance.

A preferred nucleotide analogue is LNA, such as oxy-LNA (such as beta-D-oxy-LNA, and alpha-L-oxy-LNA), and/or amino-LNA (such as beta-D-amino-LNA and alpha-L-amino-LNA) and/or thio-LNA (such as beta-D-thio-LNA and alpha-L-thio-LNA) and/or ENA (such as beta-D-ENA and alpha-L-ENA). In various embodiments the LNA units are beta-D-oxy-LNA.

In some embodiments the nucleotide analogues present within the oligomer of the invention (such as in regions A and C mentioned herein) are independently selected from, for example: 2'-O-alkyl-RNA units, 2'-amino-DNA units, 2'-fluoro-DNA units, LNA units, arabino nucleic acid (ANA) units, 2'-fluoro-ANA units, HNA units, INA (intercalating nucleic acid -Christensen, 2002. Nucl. Acids. Res. 2002 30: 4918-4925,) units and 2'MOE units. In various embodiments there is only one of the above types of nucleotide analogues present in the oligomer of the invention, or contiguous nucleobase sequence thereof.

In various embodiments the nucleotide analogues are 2'-O-methoxyethyl-RNA (2'MOE), 2'-fluoro-DNA monomers or LNA nucleotide analogues, and as such the oligonucleotide of the invention may comprise nucleotide analogues which are independently selected from these three types of analogue, or may comprise only one type of analogue selected from the three types. In various embodiments at least one of said nucleotide analogues is 2'-MOE-RNA, such as 2, 3, 4, 5, 6, 7, 8, 9 or 10 2'-MOE-RNA nucleotide units. In various embodiments at least one of said nucleotide analogues is 2'-fluoro DNA, such as 2, 3, 4, 5, 6, 7, 8, 9 or 10 2'-fluoro-DNA nucleotide units.

In various embodiments, the oligomer according to the invention comprises at least one Locked Nucleic Acid (LNA) unit, such as 1, 2, 3, 4, 5, 6, 7, or 8 LNA units, such as between 3 - 7 or 4 to 8 LNA units, or 3, 4, 5, 6 or 7 LNA units. In various embodiments, all the nucleotide analogues are LNA. In some embodiments, the oligomer may comprise both beta-D-oxy-LNA, and one or more of the following LNA units: thio-LNA, amino-LNA, oxy-LNA, and/or ENA in either the beta-D or alpha-L configurations or combinations thereof. In various embodiments all LNA cytosine units are 5'methyl-Cytosine. In various embodiments of the invention, the oligomer may comprise both LNA and DNA units. Preferably the combined total of LNA and DNA units is 10-25, preferably 10-20, even more preferably 12-16. In various embodiments of the invention, the nucleobase sequence of the oligomer, such as the contiguous nucleobase sequence consists of at least one LNA and the remaining nucleotide units are DNA units. In various embodiments the oligomer comprises only LNA nucleotide analogues and naturally occurring nucleotides (such as RNA or DNA, most preferably DNA nucleotides), optionally with modified linkage groups such as phosphorothioate.

In various embodiments, at least one of the nucleobases present in the oligomer has a modified base selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

### LNA

The term "LNA" refers to a bicyclic nucleotide analogue, known as "Locked Nucleic Acid". It may refer to an LNA monomer, or, when used in the context of an "LNA oligonucleotide" or LNA "oligomer" refers to an oligomer containing one or more such bicyclic nucleotide analogues.

The LNA used in the oligomers of the invention preferably has the structure of the general formula I 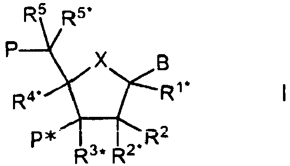 wherein X is selected from -O-, -S-, -N(R^{N*})-, -C(R⁶R^{6*})-;
B is selected from hydrogen, optionally substituted C₁₋₄-alkoxy, optionally substituted C₁₋₄-alkyl, optionally substituted C₁₋₄-acyloxy, nucleobases, DNA intercalators, photochemically active groups, thermochemically active groups, chelating groups, reporter groups, and ligands;
P designates the radical position for an internucleotide linkage to a succeeding monomer, or a 5'-terminal group, such internucleotide linkage or 5'-terminal group optionally including the substituent R⁵ or equally applicable the substituent R^{5*};
P* designates an internucleotide linkage to a preceding monomer, or a 3'-terminal group;
R^{4*} and R^{2*} together designate a biradical consisting of 1-4 groups/atoms selected from ₋C(R^{a}R^{b})-, -C(R^{a})=C(R^{b})-, -C(R^{a})=N-, -O-, -Si(R^{a})₂-, -S-, -SO₂-, - N(R^{a})-, and >C=Z,
wherein Z is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂), and
each of the substituents R¹*, R², R³, R⁵, R⁵*, R⁶ and R⁶*, which are present is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-aminocarbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected from hydrogen and C₁₋₄-alkyl, and where two adjacent (non-geminal) substituents may designate an additional bond resulting in a double bond; and R^{N}*, when present and not involved in a biradical, is selected from hydrogen and C₁₋₄-alkyl; and basic salts and acid addition salts thereof;
In various embodiments R⁵* is selected from H, -CH₃, -CH₂-CH₃,- CH₂-O-CH₃, and -CH=CH₂.
In various embodiments, R⁴* and R²* together designate a biradical selected from -C(R^{a}R^{b})-O-, -C(R^{a}R^{b})-C(R^{c}R^{d})-O-, -C(R^{a}R^{b})-C(R^{c}R^{d})-C(R^{e}R^{f})-O-, -C(R^{a}R^{b})-O-C(R^{c}R^{d})-, -C(R^{a}R^{b})-O-C(R^{c}R^{d})-O-, -C(R^{a}R^{b})-C(R^{c}R^{d})-, -C(R^{a}R^{b})-C(R^{c}R^{d})-C(R^{e}R^{f})-, - C(R^{a})=C(R^{b})-C(R^{c}R^{d})-, -C(R^{a}R^{b})-N(R^{c})-, -C(R^{a}R^{b})-C(R^{c}R^{d})- N(R^{e})-, -C(R^{a}R^{b})-N(R^{c})-O-, and -C(R^{a}R^{b})-S-, -C(R^{a}R^{b})-C(R^{c}R^{d})-S-, wherein R^{a}, R^{b}, R^{c}, R^{d}, R^{e}, and R^{f} each is independently selected from hydrogen, optionally substituted C₁₋₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-aminocarbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂),
In a further embodiment R⁴* and R²* together designate a biradical selected from -CH₂-O-, -CH₂-S-, -CH₂-NH-, -CH₂-N(CH₃)-, -CH₂-CH₂-O-, -CH₂-CH(CH₃)-, -CH₂-CH₂-S-, - CH₂-CH₂-NH-, -CH₂-CH₂-CH₂-, -CH₂-CH₂-CH₂-O-, -CH₂-CH₂-CH(CH₃)-, -CH=CH-CH₂-, -CH₂-O-CH₂-O-, -CH₂-NH-O-, -CH₂-N(CH₃)-O-, -CH₂-O-CH₂-, -CH(CH₃)-O-, -CH(CH₂-O-CH₃)-O-.

For all chiral centers, asymmetric groups may be found in either R or S orientation.

Preferably, the LNA used in the oligomer of the invention comprises at least one LNA unit according to any of the formulas 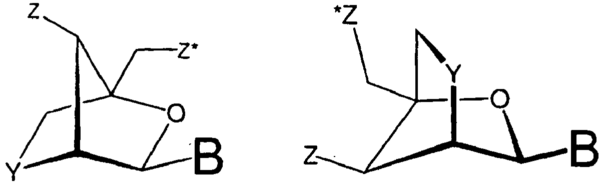 wherein Y is -O-, -O-CH₂- ,-S-, -NH-, or N(R^{H}); Z and Z* are independently selected among an internucleotide linkage, a terminal group or a protecting group; B constitutes a natural or non-natural nucleotide base moiety, and R^{H} is selected from hydrogen and C₁₋₄-alkyl.

Specifically preferred LNA units are shown in scheme 2:

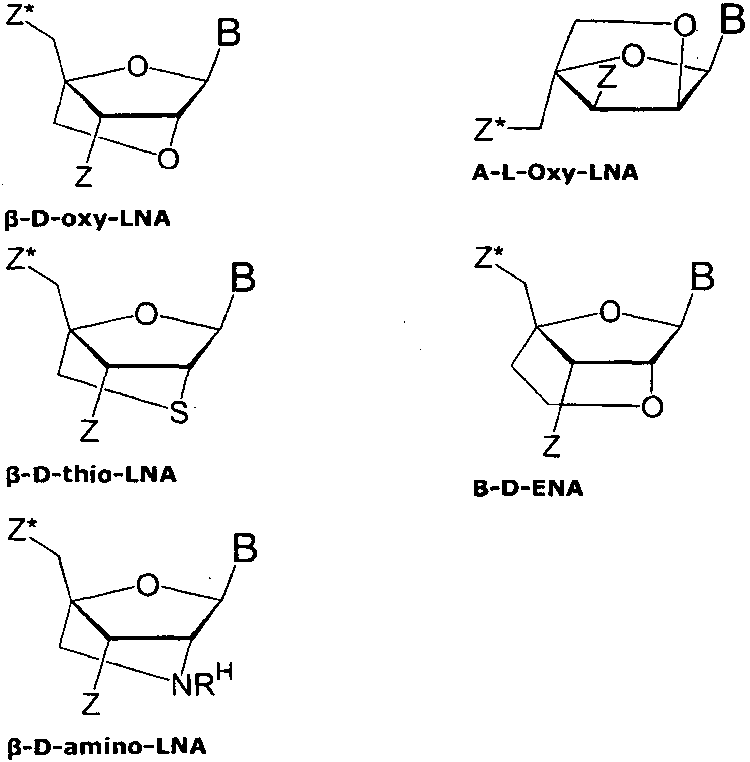

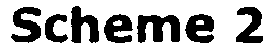

The term "thio-LNA" comprises a locked nucleobase (LNA) in which Y in the general formula above is selected from S or -CH₂-S-. Thio-LNA can be in both beta-D and alpha-L-configuration.

The term "amino-LNA" comprises a locked nucleobases (LNA) in which Y in the general formula above is selected from -N(H)-, N(R)-, CH₂-N(H)-, and -CH₂-N(R)- where R is selected from hydrogen and C₁₋₄-alkyl. Amino-LNA can be in both beta-D and alpha-L-configuration.

The term "oxy-LNA" comprises a locked nucleobase (LNA) in which Y in the general formula above represents -O- or -CH₂-O-. Oxy-LNA can be in both beta-D and alpha-L-configuration.

The term "ENA" comprises a locked nucleobase (LNA) in which Y in the general formula above is -CH₂-O- (where the oxygen atom of -CH₂-O- is attached to the 2'-position relative to the base B).

In a preferred embodiment LNA is selected from beta-D-oxy-LNA, alpha-L-oxy-LNA, beta-D-amino-LNA and beta-D-thio-LNA, in particular beta-D-oxy-LNA.

In the present context, the term "C₁₋₄-alkyl" is intended to mean a linear or branched saturated hydrocarbon chain wherein the chain has from one to four carbon atoms, such as methyl, ethyl, n-propyl, isopropyl, n-butyl, isobutyl, sec-butyl and tert-butyl.

### RNAse H recruitment

It is recognised that an oligomeric compound may function via non RNase mediated degradation of target mRNA, such as by steric hindrance of translation, or other methods, however, the preferred oligomers of the invention are capable of recruiting an endoribonuclease (RNase), such as RNase H.

In various embodiments, that the oligomer, or contiguous nucleobase sequence, comprises of a region of at least 6, such as at least 7 consecutive nucleotide or nucleobase units, such as at least 8 or at least 9 consecutive nucleobase units (residues), including 7, 8, 9, 10, 11, 12, 13, 14, 15 or 16 consecutive nucleotides or nucleobases, which, when formed in a duplex with the complementary target RNA is capable of recruiting RNase. The contiguous sequence which is capable of recruiting RNAse may be region B as referred to in the context of a gapmer as described herein. In various embodiments the size of the contiguous sequence which is capable of recruiting RNAse, such as region B, may be higher, such as 10, 11, 12, 13, 14, 15, 16, 17, 18, 19 or 20 nucleobase units.

EP 1 222 309 provides *in vitro* methods for determining RNaseH activity, which may be used to determine the ability to recruit RNaseH. A oligomer is deemed capable of recruiting RNase H if, when provided with the complementary RNA target, it has an initial rate, as measured in pmol/l/min, of at least 1 %, such as at least 5%, such as at least 10% or less than 20% of the equivalent DNA only oligonucleotide, with no 2' substitutions, with phosphorothioate linkage groups between all nucleotides in the oligonucleotide, using the methodology provided by Example 91 - 95 of EP 1 222 309.

In various embodiments, an oligomer is deemed essentially incapable of recruiting RNaseH if, when provided with the complementary RNA target, and RNaseH, the RNaseH initial rate, as measured in pmol/l/min, is less than 1%, such as less than 5%,such as less than 10% or less than 20% of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

In other embodiments, an oligomer is deemed capable of recruiting RNaseH if, when provided with the complementary RNA target, and RNaseH, the RNaseH initial rate, as measured in pmol/l/min, is at least 20%, such as at least 40 %, such as at least 60 %, such as at least 80 % of the initial rate determined using the equivalent DNA only oligonucleotide (i.e. having the same base sequence, but containing only DNA units), with no 2' substitutions, with phosphorothioate linkage groups between all nucleotides in the oligonucleotide, using the methodology provided by Example 91 - 95 of EP 1 222 309.

Typically the region of the oligomer which forms the consecutive nucleobase sequence, when formed in a duplex with the complementary target RNA, is capable of recruiting RNase consists of nucleotide units which form a DNA/RNA like duplex with the RNA target - and may include both DNA units and LNA units which are in the alpha-L configuration, particularly preferred being alpha-L-oxy LNA.

The oligomer of the invention may comprise a nucleobase sequence which comprises both nucleotides and nucleotide analogues, and may be in the form of a gapmer, a headmer or a mixmer.

A headmer is defined by a contiguous stretch of non-RNase recruiting nucleotide analogues at the 5'-end followed by a contiguous stretch of DNA or modified nucleotide (analogues) units recognizable and cleavable by the RNAse towards the 3'-end (such as at least 7 such nucleotides), and a tailmer is defined by a contiguous stretch of DNA or modified nucleotides (analogues) recognizable and cleavable by the RNase at the 5'-end (such as at least 7 such nucleotides), followed by a contiguous stretch of non-RNase recruiting nucleotide analogues towards the 3'-end. Other "chimeric" oligomers according to the invention, called "mixmers" consist of an alternating composition of i) DNA or modified nucleotides recognizable and cleavable by RNase and ii) non-RNase recruiting nucleotide analogues. Some nucleotide analogues may also be able to mediate RNase, such as RNaseH, binding and cleavage. Since a-L-LNA recruits RNase activity to a certain extent, smaller regions (e.g. gaps - or region B) of DNA or modified nucleotides recognizable and cleavable by the RNase for the gapmer construct might be required, and more flexibility in the mixmer construction might be introduced.

### Gapmer Design

Preferably, the oligomer of the invention is a gapmer. A gapmer oligomer is an oligomer which comprises a contiguous stretch of nucleotides or nucleobases which is capable of recruiting an RNAse, such as RNAseH, such as a region of at least 6 or 7 DNA nucleotides, referred to herein in as region B, wherein region B is flanked both 5' and 3' by regions of nucleotide analogues, such as affinity enhancing nucleotide analogues, such as between 1 - 6 nucleotide analogues 5' and 3' to the contiguous stretch of nucleotides which is capable of recruiting RNAse - these regions are referred to as regions A and C respectively.

Preferably the gapmer comprises a (poly)nucleotide sequence of formula (5' to 3'), A-B-C, or optionally A-B-C-D or D-A-B-C, wherein; region A (5' region) consists or comprises of at least one nucleotide analogue, such as at least one LNA unit, such as between 1-6 nucleotide analogues, such as LNA units, and; region B consists or comprises of at least five consecutive nucleotides or nucleobases which are capable of recruiting RNAse (when formed in a duplex with a complementary RNA molecule, such as the mRNA target), such as DNA nucleotides, and; region C (3'region) consists or comprises of at least one nucleotide analogue, such as at least one LNA unit, such as between 1-6 nucleotide analogues, such as LNA units, and; region D, when present consists or comprises of 1, 2 or 3 nucleotide units, such as DNA nucleotides.

In various embodiments, region A consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units; and/or region C consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units.

In various embodiments B consists or comprises of 5, 6, 7, 8, 9, 10, 11 or 12 consecutive nucleotides or nucleobases which are capable of recruiting RNAse, or between 6-10, or between 7-9, such as 8 consecutive nucleotides or nucleobases which are capable of recruiting RNAse. In various embodiments region B consists or comprises at least one DNA nucleotide unit, such as 1-12 DNA units, preferably between 4-12 DNA units, more preferably between 6-10 DNA units, such as between 7-10 DNA units, most preferably 8, 9 or 10 DNA units.

In various embodiments region A consist of 3 or 4 nucleotide analogues, such as LNA, region B consists of 7, 8, 9 or 10 DNA units, and region C consists of 3 or 4 nucleotide analogues, such as LNA. Such designs include (A-B-C) 3-10-3, 3-10-4, 4-10-3, 3-9-3, 3-9-4, 4-9-3, 3-8-3, 3-8-4, 4-8-3, 3-7-3, 3-7-4, 4-7-3, and may further include region D, which may have one or 2 nucleotide units, such as DNA units.

Further gapmer designs are disclosed in WO2004/046160 US provisional application, 60/977409, refers to 'shortmer' gapmer oligomers, which, in various embodiments may be the gapmer oligomer according to the present invention.

In various embodiments the oligomer is consisting of a contiguous nucleobase sequence of a total of 10, 11, 12, 13 or 14 nucleobase units, wherein the contiguous nucleobase sequence is of formula (5' - 3'), A-B-C, or optionally A-B-C-D or D-A-B-C, wherein; A consists of 1, 2 or 3 nucleotide analogue units, such as LNA units; B consists of 7, 8 or 9 contiguous nucleotide or nucleobase units which are capable of recruiting RNAse when formed in a duplex with a complementary RNA molecule (such as a mRNA target); and C consists of 1, 2 or 3 nucleotide analogue units, such as LNA units. When present, D consists of a single DNA unit.

In various embodiments A consists of 1 LNA unit. In various embodiments A consists of 2 LNA units. In various embodiments A consists of 3 LNA units. In various embodiments C consists of 1 LNA unit. In various embodiments C consists of 2 LNA units. In various embodiments C consists of 3 LNA units. In various embodiments B consists of 7 nucleotide units. In various embodiments B consists of 8 nucleotide units. In various embodiments B consists of 9 nucleotide units. In various embodiments B comprises of between 1 - 9 DNA units, such as 2, 3, 4, 5, 6, 7 or 8 DNA units. In various embodiments B consists of DNA units. In various embodiments B comprises of at least one LNA unit which is in the alpha-L configuration, such as 2, 3, 4, 5, 6, 7, 8 or 9 LNA units in the alpha-L-configuration. In various embodiments B comprises of at least one alpha-L-oxy LNA unit or wherein all the LNA units in the alpha-L- configuration are alpha-L-oxy LNA units. In various embodiments the number of nucleotides present in A-B-C are selected from the group consisting of (nucleotide analogue units - region B - nucleotide analogue units): 1-8-1, 1-8-2, 2-8-1, 2-8-2, 3-8-3, 2-8-3, 3-8-2, 4-8-1, 4-8-2, 1-8-4, 2-8-4, or; 1-9-1, 1-9-2, 2-9-1, 2-9-2, 2-9-3, 3-9-2, 1-9-3, 3-9-1, 4-9-1, 1-9-4; or; 1-10-1, 1-10-2, 2-10-1, 2-10-2, 1-10-3, 3-10-1. In various embodiments the number of nucleotides in A-B-C are selected from the group consisting of: 2-7-1, 1-7-2, 2-7-2, 3-7-3, 2-7-3, 3-7-2, 3-7-4, and 4-7-3. In various embodiments both A and C consists of two LNA units each, and B consists of 8 or 9 nucleotide units, preferably DNA units.

Other gapmer designs include those where region A and or C consists of 3, 4, 5 or 6 nucleotide analogues, such as nucleotide analogues selected from the group consisting of 2'-O-methoxyethyl-RNA (2'MOE) and 2'-fluoro-DNA monomers, and region B consists of 8, 9, 10, 11 or 12 nucleotides, such as DNA units (or nudeobases), such as 5-10-5 and 4-12-4 gapmer designs. Further gapmers designs are disclosed in WO 2007/146511A2,

### Linkage groups

The terms "linkage group" or "internucleotide linkage" are intended to mean a group capable of covalently coupling together two nucleotides or nucleobases, two nucleotide analogues, and a nucleotide and a nucleotide analogue, etc. Specific and preferred examples include phosphate groups and phosphorothioate groups.

The nucleobases of the oligomer of the invention or contiguous nucleobase sequence thereof are coupled together via linkage groups. Suitably each nucleobase is linked to the 3' adjacent nucleobase via a linkage group.

Suitable linkage groups include those listed within WO2007/031091, for example the linkage groups listed on the first paragraph of page 34 of WO2007/031091.

It is, in various embodiments, preferred to modify the linkage group from its normal phosphodiester to one that is more resistant to nuclease attack, such as phosphorothioate or boranophosphate - these two, being cleavable by RNase H, also allow that route of antisense inhibition in reducing the expression of the target gene.

Suitable sulphur (S) containing linkage groups as provided herein may be preferred. Phosphorothioate linkage groups are also preferred, particularly for the gap region (B) of gapmers. Phosphorothioate linkages may also be used for the flanking regions (A and C, and for linking A or C to D, and within region D, as appropriate).

Regions A, B and C, may however comprise linkage groups other than phosphorothioate, such as phosphodiester linkages, particularly, for instance when the use of nucleotide analogues protects the linkage groups within regions A and C from endo-nuclease degradation - such as when regions A and C comprise LNA nucleotides.

The linkage groups in the oligomer may be phosphodiester, phosphorothioate or boranophosphate so as to allow RNase H cleavage of targeted RNA. Phosphorothioate is preferred, for improved nuclease resistance and other reasons, such as ease of manufacture.

In one aspect of the oligomer of the invention, the nucleotides and/or nucleotide analogues are linked to each other by means of phosphorothioate groups.

It is recognised that the inclusion of phosphodiester linkages, such as one or two linkages, into an otherwise phosphorothioate oligomer, particularly between or adjacent to nucleotide analogue units (typically in region A and or C) can modify the bioavailability and/or bio-distribution of an oligomer - see WO2008/053314.

In some embodiments, such as the embodiments referred to above, where suitable and not specifically indicated, all remaining linkage groups are either phosphodiester or phosphorothioate, or a mixture thereof.

In some embodiments all the internucleotide linkage groups are phosphorothioate.

When referring to specific gapmer oligonucleotide sequences, such as those provided herein it will be understood that, in various embodiments, when the linkages are phosphorothioate linkages, alternative linkages, such as those disclosed herein may be used, for example phosphate (phosphodiester) linkages may be used, particularly for linkages between nucleotide analogues, such as LNA, units. Likewise, when referring to specific gapmer oligonucleotide sequences, such as those provided herein, when the C residues are annotated as 5'methyl modified cytosine, in various embodiments, one or more of the Cs present in the oligomer may be unmodified C residues.

### Oligomeric Compounds

The oligomers of the invention may be selected from the group consisting of: SEQ ID NO 133 - 152, as shown in table 2.

**Table 2 Oligomer designs** SEQ ID NOS: 133-152, upper case letters indicates nucleotide analogue units and subscript "s" represents phosphorothiate linkage. Lower case letters represent nucleotide (DNA) units. Absence of "s" (if any) indicates a phosphodiester linkage.

| **Table 2 - Oligomer designs** | | |
|---|---|---|
| **SEQ ID NO** | **Sequence (5'-3')** | **Length (bases)** |
| SEQ ID NO: 133 | **G_{S}A_{S}A_{S}**a_{S}g_{S}c_{S}t_{S}g_{S}a_{S}t_{S}g_{S}g_{S}a_{S}**C_{S}C_{S}A** | 16 |
| SEQ ID NO: 134 | **C_{S}A_{S}G_{S}**a_{S}C_{S}t_{S}t_{S}a_{S}a_{S}a_{S}g_{S}a_{S}t_{S}**G_{S}G_{S}C** | 16 |
| SEQ ID NO: 135 | **C_{S}A_{S}G_{S}**a_{S}a_{S}t_{S}c_{S}c_{S}a_{S}c_{S}t_{S}g_{S}g_{S}**T_{S}G_{S}A** | 16 |
| SEQ ID NO: 136 | **G_{S}C_{S}A_{S}**C_{S}t_{S}g_{S}C_{S}C_{S}a_{S}t_{S}t_{S}t_{S}t_{S}**A_{S}G_{S}C** | 16 |
| SEQ ID NO: 137 | **G_{S}T_{S}A_{S}**a_{S}t_{S}a_{S}g_{S}C_{S}C_{S}a_{S}a_{S}g_{S}a_{S}**A_{S}T_{S}T** | 16 |
| SEQ ID NO: 138 | **A_{S}C_{S}T_{S}**C_{S}t_{S}g_{S}C_{S}t_{S}t_{S}g_{S}t_{S}g_{S}g_{S}**T_{S}C_{S}C** | 16 |
| SEQ ID NO: 139 | **C_{S}C_{S}A_{S}**c_{S}c_{S}a_{S}g_{S}c_{S}t_{S}t_{S}c_{S}t_{S}a_{S}**C_{S}A_{S}A** | 16 |
| SEQ ID NO: 140 | **G_{S}A_{S}G_{S}**t_{S}c_{S}c_{S}a_{S}a_{S}a_{S}g_{S}a_{S}C_{S}a_{S}**G_{S}T_{S}T** | 16 |
| SEQ ID NO: 141 | **A_{S}C_{S}Cₛ**cₛa_{S}c_{S}tₛtₛg_{S}g_{S}c_{S}a_{S}g_{S}**A_{S}C_{S}C** | 16 |
| SEQ ID NO: 142 | **G_{S}C_{S}A**_{S}c_{S}a_{S}a_{S}a_{S}c_{S}a_{S}a_{S}t_{S}g_{S}g_{S}**A_{S}A_{S}T** | 16 |
| SEQ ID NO: 143 | **G_{S}C_{S}A_{S}**g_{S}c_{S}t_{S}a_{S}c_{S}t_{S}c_{S}t_{S}t_{S}t_{S}**G_{S}G_{S}A** | 16 |
| SEQ ID NO: 144 | **C_{S}T_{S}C_{S}**c_{S}c_{S}t_{S}c_{S}a_{S}g_{S}c_{S}t_{S}t_{S}c_{S}**A_{S}A_{S}T** | 16 |
| SEQ ID NO: 145 | **G_{S}C_{S}A_{S}**g_{S}t_{S}c_{S}t_{S}c_{S}a_{S}t_{S}t_{S}c_{S}c_{S}**A_{S}A_{S}G** | 16 |
| SEQ ID NO: 146 | **T_{S}A_{S}T**_{S}c_{S}c_{S}a_{S}c_{S}c_{S}a_{S}g_{S}a_{S}g_{S}t_{S}**G_{S}AₛA** | 16 |
| SEQ ID NO: 147 | **C_{S}A_{S}T_{S}**c_{S}c_{S}a_{S}t_{S}g_{S}a_{S}g_{S}g_{S}t_{S}c_{SC}**c_{S}T_{S}G** | 16 |
| SEQ ID NO: 148 | **C_{S}C_{S}A_{S}**t_{S}c_{S}t_{S}t_{S}g_{S}t_{S}g_{S}a_{S}t_{S}c_{S}**C_{S}A_{S}T** | 16 |
| SEQ ID NO: 149 | **A_{S}A_{S}G_{S}**c_{S}a_{S}a_{S}g_{S}c_{S}a_{S}a_{S}a_{S}g_{S}t_{S}**C_{S}A_{S}G** | 16 |
| SEQ ID NO: 150 | **G_{S}A_{S}A_{S}**a_{S}t_{S}t_{S}g_{S}c_{S}t_{S}g_{S}t_{S}a_{S}g_{S}**C_{S}A_{S}G** | 16 |
| SEQ ID NO: 151 | **G_{S}T_{S}G_{S}**t_{S}t_{S}t_{S}a_{S}c_{S}a_{S}c_{S}c_{S}a_{S}**T_{S}T_{S}A** | 16 |
| SEQ ID NO: 152 | **A_{S}A_{S}C_{S}**a_{S}t_{S}g_{S}a_{S}a_{S}a_{S}t_{S}a_{S}g_{S}a_{S}**T_{S}C_{S}C** | 16 |

### Conjugates

In the context the term "conjugate" is intended to indicate a heterogenous molecule formed by the covalent attachment ("conjugation") of the oligomer as described herein to one or more non-nucleotide/non- nucleotide-analogue (*i.e.* non-nucleobase), or non-polynucleotide moieties. Examples of non-nucleotide or non- polynucleotide moieties include macromolecular agents such as proteins, fatty acid chains, sugar residues, glycoproteins, polymers, or combinations thereof. Typically proteins may be antibodies for a target protein. Typical polymers may be polyethylene glycol.

Therefore, in various embodiments, the oligomer of the invention may comprise both a polynucleotide region, *i.e.* a nucleotide/nucleobase region, which typically consists of a contiguous sequence of nucleobases, and a further non-nucleotide region. When referring to the oligomer of the invention consisting of a contiguous nucleobase sequence, the compound may comprise non-nucleotide/non-nucleobase components, such as a conjugate component.

In various embodiments of the invention the oligomeric compound is linked to ligands/conjugates, which may be used, e.g. to increase the cellular uptake of oligomeric compounds. WO2007/031091 provides suitable ligands and conjugates.

The invention also provides for a conjugate comprising the compound according to the invention as herein described, and at least one non-nucleotide or non-polynucleotide moiety covaientiy attached to said compound. Therefore, in various embodiments where the compound of the invention consists of a specified nucleic acid or nucleobase sequence, as herein disclosed, the compound may also comprise at least one non-nucleotide or non-polynucleotide moiety (*e.g*. not comprising one or more nucleotides or nucleotide analogues) covalently attached to said compound.

Conjugates may enhance the activity, cellular distribution or cellular uptake of the oligomer of the invention. Such moieties include, but are not limited to, antibodies, polypeptides, lipid moieties such as a cholesterol moiety, cholic acid, a thioether, *e.g*. Hexyl-s-tritylthiol, a thiocholesterol, an aliphatic chain, *e.g*., dodecandiol or undecyl residues, a phospholipids, *e.g*., di-hexadecyl-rac-glycerol or triethylammonium 1,2-di-o-hexadecyl-rac-glycero-3-h-phosphonate, a polyamine or a polyethylene glycol chain, an adamantane acetic acid, a palmityl moiety, an octadecylamine or hexylamino-carbonyl-oxycholesterol moiety.

The oligomers of the invention may also be conjugated to active drug substances, for example, aspirin, ibuprofen, a sulfa drug, an antidiabetic, an antibacterial or an antibiotic.

In certain embodiments the conjugated moiety is a sterol, such as cholesterol.

In various embodiments, the conjugated moiety comprises or consists of a positively charged polymer, such as a positively charged peptides of, for example between 1 -50, such as 2 - 20 such as 3 - 10 amino acid residues in length, and/or polyalkylene oxide such as polyethylglycol(PEG) or polypropylene glycol - see WO 2008/034123. Suitably the positively charged polymer, such as a polyalkylene oxide may be attached to the oligomer of the invention via a linker such as the releasable inker described in WO 2008/034123.

### Activated oligomers

The term "activated oligomer," as used herein, refers to an oligomer of the invention that is covalently linked (i.e., functionalized) to at least one functional moiety that permits covalent linkage of the oligomer to one or more conjugated moieties, i.e., moieties that are not themselves nucleic acids or monomers, to form the conjugates herein described. Typically, a functional moiety will comprise a chemical group that is capable of covalently bonding to the oligomer via, e.g., a 3'-hydroxyl group or the exocyclic NH2 group of the adenine base, a spacer that is preferably hydrophilic and a terminal group that is capable of binding to a conjugated moiety (e.g., an amino, sulfhydryl or hydroxyl group). In some embodiments, this terminal group is not protected, e.g., is an NH2 group. In other embodiments, the terminal group is protected, for example, by any suitable protecting group such as those described in "Protective Groups in Organic Synthesis" by Theodora W Greene and Peter G M Wuts, 3rd edition (John Wiley & Sons, 1999). Examples of suitable hydroxyl protecting groups include esters such as acetate ester, aralkyl groups such as benzyl, diphenylmethyl, or triphenylmethyl, and tetrahydropyranyl. Examples of suitable amino protecting groups include benzyl, alpha-methylbenzyl, diphenylmethyl, triphenylmethyl, benzyloxycarbonyl, tert-butoxycarbonyl, and acyl groups such as trichloroacetyl or trifluoroacetyl. In some embodiments, the functional moiety is self-cleaving. In other embodiments, the functional moiety is biodegradable. See e.g., U.S. Patent No. 7,087,229.

In some embodiments, oligomers of the invention are functionalized at the 5' end in order to allow covalent attachment of the conjugated moiety to the 5' end of the oligomer. In other embodiments, oligomers of the invention can be functionalized at the 3' end. In still other embodiments, oligomers of the invention can be functionalized along the backbone or on the heterocyclic base moiety. In yet other embodiments, oligomers of the invention can be functionalized at more than one position independently selected from the 5' end, the 3' end, the backbone and the base.

In some embodiments, activated oligomers of the invention are synthesized by incorporating during the synthesis one or more monomers that is covalently attached to a functional moiety. In other embodiments, activated oligomers of the invention are synthesized with monomers that have not been functionalized, and the oligomer is functionalized upon completion of synthesis. In some embodiments, the oligomers are functionalized with a hindered ester containing an aminoalkyl linker, wherein the alkyl portion has the formula (CH2)w, wherein w is an integer ranging from 1 to 10, preferably about 6, wherein the alkyl portion of the alkylamino group can be straight chain or branched chain, and wherein the functional group is attached to the oligomer via an ester group (-O-C(O)-(CH2)wNH).

In other embodiments, the oligomers are functionalized with a hindered ester containing a (CH2)w-sulfhydryl (SH) linker, wherein w is an integer ranging from 1 to 10, preferably about 6, wherein the alkyl portion of the alkylamino group can be straight chain or branched chain, and wherein the functional group attached to the oligomer via an ester group (-O-C(O)-(CH2)wSH)

In some embodiments, sulfhydryl-activated oligonucleotides are conjugated with polymer moieties such as polyethylene glycol or peptides (via formation of a disulfide bond).

Activated oligomers containing hindered esters as described above can be synthesized by any method known in the art, and in particular by methods disclosed in PCT Publication No. WO 2008/034122 and the examples therein,

In still other embodiments, the oligomers of the invention are functionalized by introducing sulfhydryl, amino or hydroxyl groups into the oligomer by means of a functionalizing reagent substantially as described in U.S. Patent Nos. 4,962,029 and 4,914,210, i.e., a substantially linear reagent having a phosphoramidite at one end linked through a hydrophilic spacer chain to the opposing end which comprises a protected or unprotected sulfhydryl, amino or hydroxyl group. Such reagents primarily react with hydroxyl groups of the oligomer. In some embodiments, such activated oligomers have a functionalizing reagent coupled to a 5'-hydroxyl group of the oligomer. In other embodiments, the activated oligomers have a functionalizing reagent coupled to a 3'-hydroxyl group. In still other embodiments, the activated oligomers of the invention have a functionalizing reagent coupled to a hydroxyl group on the backbone of the oligomer. In yet further embodiments, the oligomer of the invention is functionalized with more than one of the functionalizing reagents as described in U.S. Patent Nos. 4,962,029 and 4,914,210. Methods of synthesizing such functionalizing reagents and incorporating them into monomers or oligomers are disclosed in U.S. Patent Nos. 4,962,029 and 4,914,210.

In some embodiments, the 5'-terminus of a solid-phase bound oligomer is functionalized with a dienyl phosphoramidite derivative, followed by conjugation of the deprotected oligomer with, e.g., an amino acid or peptide via a Diels-Alder cycloaddition reaction.

In various embodiments, the incorporation of monomers containing 2'-sugar modifications, such as a 2'-carbamate substituted sugar or a 2'-(O-pentyl-N-phthalimido)-deoxyribose sugar into the oligomer facilitates covalent attachment of conjugated moieties to the sugars of the oligomer. In other embodiments, an oligomer with an amino-containing linker at the 2'-position of one or more monomers is prepared using a reagent such as, for example, 5'-dimethoxytrityl-2'-O-(e-phthalimidylaminopentyl)-2'-deoxyadenosine-3'-- N,N-dilsopropyl-cyanoethoxy phosphoramidite. See, e.g., Manoharan, et al., Tetrahedron Letters, 1991, 34, 7171.

In still further embodiments, the oligomers of the invention may have amine-containing functional moieties on the nucleobase, including on the N6 purine amino groups, on the exocyclic N2 of guanine, or on the N4 or 5 positions of cytosine. In one embodiment, such functionalization may be achieved by using a commercial reagent that is already functionalized in the oligomer synthesis.

Some functional moieties are commercially available, for example, heterobifunctional and homobifunctional linking moieties are available from the Pierce Co. (Rockford, III.). Other commercially available linking groups are 5'-Amino-Modifier C6 and 3'-Amino-Modifier reagents, both available from Glen Research Corporation (Sterling, Va.). 5'-Amino-Modifier C6 is also available from ABI (Applied Biosystems Inc., Foster City, Calif.) as Aminolink-2, and 3'-Amino-Modifier is also available from Clontech Laboratories Inc. (Palo Alto, Calif.).

### Compositions

The oligomer of the invention may be used in pharmaceutical formulations and compositions. Suitably, such compositions comprise a pharmaceutically acceptable diluent, carrier, salt or adjuvant. WO2007/031091 provides suitable and preferred pharmaceutically acceptable diluents, carriers and adjuvants. Suitable dosages, formulations, administration routes, compositions, dosage forms, combinations with other therapeutic agents, pro-drug formulations are also provided in WO2007/031091

As used herein, the term "pharmaceutically acceptable salts" refers to salts that retain the desired biological activity of the herein identified compounds and exhibit minimal undesired toxicological effects. Non-limiting examples of such salts can be formed with organic amino acid and base addition salts formed with metal cations such as zinc, calcium, bismuth, barium, magnesium, aluminum, copper, cobalt, nickel, cadmium, sodium, potassium, and the like, or with a cation formed from ammonia, /V,/V-dibenzylethylene-diamine, D-glucosamine, tetraethylammonium, or ethylenediamine; or (c) combinations of (a) and (b); e.g., a zinc tannate salt or the like.

### Applications

The oligomers of the invention may be utilized as research reagents for, for example, diagnostics, therapeutics and prophylaxis.

In research, such oligomers may be used to specifically inhibit the expression or synthesis of beta-catenin protein (typically by degrading or inhibiting the mRNA and thereby prevent protein formation) in cells and experimental animals thereby facilitating functional analysis of the target or an appraisal of its usefulness as a target for therapeutic intervention.

In diagnostics the oligomers may be used to detect and quantitate beta-catenin expression in cells and tissues by northern blotting, in-situ hybridisation or similar techniques.

For therapeutics, an animal or a human, suspected of having a disease or disorder, which can be treated by modulating the expression of beta-catenin is treated by administering oligomeric compounds, such as an effective amount of an oligomer (or conjugate thereof) in accordance with this invention. Further provided are methods of treating a mammal, such as treating a human, suspected of having or being prone to a disease or condition, associated with expression of beta-catenin by administering a therapeutically or prophylactically effective amount of one or more of the oligomers or compositions of the invention.

The invention also provides for the use of the compounds or conjugate of the invention as described for the manufacture of a medicament for the treatment of a disorder as referred to herein, or for a method of the treatment of as a disorder as referred to herein.

The invention also relates to an oligomer, a composition or a conjugate as described herein for use as a medicament. Suitably, when referring to methods of the invention, the administration of the oligomer or conjugate of the invention is performed by administering an effective amount of the oligomer or conjugate, e.g. an amount which is effective to provide treatment or an amount which is effective to inhibit or down-regulate beta-catenin expression in a cell or cells.

The invention also provides for a method for treating a disorder as referred to herein said method comprising administering a compound according to the invention as herein described, and/or a conjugate according to the invention, and/or a pharmaceutical composition according to the invention to a patient in need thereof.

In various embodiments, the oligomer, or conjugate thereof, induces a desired therapeutic effect in humans through for example binding by hydrogen bonding to a target nucleic acid. The oligomer, or conjugate thereof, causes a decrease (inhibition) in the expression of a target via hydrogen bonding (hybridisation) to the mRNA of the target thereby resulting in a reduction in gene expression. It is also envisaged that the oligomeric compounds and conjugates disclosed herein may have non-therapeutic applications, such as diagnostic applications.

### Medical Indications

The disorder, as referred to herein, is in various embodiments selected from the group consisting of a hyperproliferative disorder, such as cancer, such as a cancer selected from the group consisting of colorectal cancer, hepatocellular cancer, endometrial cancer, malignant melanoma, ovarian cancer, pancreatic cancer, pituitary cancer, oesophageal cancer, lung cancer, breast cancer, kidney cancer, haematopoetic system cancer, cervical cancer, CNS cancer, bone cancer, biliary tract cancer and adrenal gland cancer

In various embodiments the disorder, as referred to herein is a cancer selected from the group consisting of colorectal cancer, hepatocellular cancer, endometrial cancer, and malignant melanoma.

In various embodiments the disorder, as referred to herein is a cancer selected from the group consisting of liver cancer and kidney cancer.

The oligomers and other compositions according to the invention can be used for the treatment of conditions associated with over expression or expression of mutated version of the beta-catenin.

The invention further provides use of a compound of the invention in the preparation or manufacture of a medicament for the treatment of a disease, disorder or condition as referred to herein.

The invention further relates to use of a compound, composition, or a conjugate as defined herein for the manufacture of a medicament for the treatment of abnormal levels of beta-catenin or expression of mutant forms of beta-catenin (such as allelic variants, such as those associated with one of the diseases referred to herein).

One aspect of the invention is directed to a method of treating a mammal suffering from or susceptible to conditions associated with abnormal levels of beta-catenin, comprising administering to the mammal and therapeutically effective amount of an oligomer targeted to beta-catenin, such as an oligomer that comprises one or more LNA units. The methods of the invention may therefore be employed for treatment or prophylaxis against diseases caused by abnormal levels of beta-catenin. Alternatively stated, in various embodiments, the invention is furthermore directed to a method for treating abnormal levels of beta-catenin, said method comprising administering a oligomer of the invention, or a conjugate of the invention or a pharmaceutical composition of the invention to a patient in need thereof.

The disease or disorder, as referred to herein, may, in various embodiments be associated with a mutation in the beta-catenin gene or a gene whose protein product is associated with or interacts with beta-catenin. Therefore, in various embodiments, the target mRNA is a mutated form of the beta-catenin sequence.

An interesting aspect of the invention is directed to the use of an oligomer (compound) as defined herein or a conjugate as defined herein for the preparation of a medicament for the treatment of a disease, disorder or condition as referred to herein.

The invention also relates to an oligomer, a composition or a conjugate as defined herein for use as a medicament.

Moreover, the invention relates to a method of treating a subject suffering from a disease or condition such as those referred to herein, said method comprising administering a compound according to the invention as herein described, and/or a conjugate according to the invention, and/or a pharmaceutical composition according to the invention to a patient in need thereof.

Suitable dosages, formulations, administration routes, compositions, dosage forms, combinations with other therapeutic agents, pro-drug formulations are also provided in WO2007/031091

. The invention also provides for a pharmaceutical composition comprising a compound or a conjugate as herein described or a conjugate, and a pharmaceutically acceptable diluent, carrier or adjuvant. WO2007/031091 provides suitable and preferred pharmaceutically acceptable diluent, carrier and adjuvants.

The methods of the invention are preferably employed for treatment or prophylaxis against diseases caused by abnormal levels of beta-catenin.

Furthermore, the invention described herein encompasses a method of preventing or treating a disease comprising a therapeutically effective amount of a beta-catenin modulating oligomer to a human in need of such therapy. The invention further encompasses the use of a short period of administration of a beta-catenin modulating oligonucleotide compound (oligomer or conjugate).

Alternatively stated, in various embodiments, the invention is furthermore directed to a method for treating abnormal levels of beta-catenin, said method comprising administering a oligomer of the invention, or a conjugate of the invention or a pharmaceutical composition of the invention to a patient in need thereof and further comprising the administration of a further agent or further active ingredient. Said further administration may be such that the further agent or further active ingredient is conjugated to the compound of the invention, is present in the pharmaceutical composition, or is administered in a separate formulation.

The invention also relates to an oligomer, a composition or a conjugate as defined herein for use as a medicament.

The invention further relates to use of a compound, composition, or a conjugate as defined herein for the manufacture of a medicament for the treatment of abnormal levels of beta-catenin or expression of mutant forms of beta-catenin (such as allelic variants, such as those associated with one of the diseases referred to herein).

Moreover, the invention relates to a method of treating a subject suffering from a disease or condition such as those referred to herein.

A patient who is in need of treatment is a patient suffering from or likely to suffer from the disease or disorder.

In various embodiments, the term 'treatment' as used herein refers to both treatment of an existing disease (*e.g.* a disease or disorder as herein referred to), or prevention of a disease, *i.e*. prophylaxis. It will therefore be recognised that treatment as referred to herein may, in various embodiments, be prophylactic.

The disease or disorder, as referred to herein, may, in various embodiments be associated with a mutation in the beta-catenin gene or a gene whose protein product is associated with or interacts with beta-catenin, such as the APC gene. Therefore, in various embodiments, the target mRNA is a mutated form of the beta-catenin sequence, for example it may comprise one or more single point mutations, such as SNPs associated with cancer.

Examples of such diseases where mutations in the beta-catenin or APC gene lead to abnormal levels of beta-catenin activity are: (1) Colorectal cancer, APC and beta-catenin are mutually mutated in more than 70% of all cases (Powell et al., Nature, 1992; Morin et al., Science, 1997; Sparks et al., Cancer Res, 1998); (2) Hepatocellular cancer, beta-catenin are mutated in more than 25% of cases (de La Coste A, PNAS, 1998); (3) Endometrial cancer, beta-catenin are mutated >10%; and (4) Malignant melanoma, Beta-catenin are mutated >10% (Rubinfeld et al., Science, 1997).

Further examples of such diseases are cancer of the ovary, pancreas, pituitary, oesophagus, lung, breast, kidney, haematopoetic system, cervix, CNS, bone, biliary tract and adrenal gland. It has been shown that mutations in the beta-catenin or APC gene are associated with these diseases (Catalogue of Somatic Mutations in Cancer available from the Sanger Institute (United Kingdom) homepage http://www.sanger.ac.uk/).

The cancer referred to in accordance with the invention, may be selected from one of the above mentioned forms of cancer.

### Further embodiments

The following embodiments also relate to the description and summary of the invention, and may be combined with the embodiments of the invention referred to therein including the embodiment referred to in the claims:
1. An antisense oligonucleotide (such as the oligomer) capable of binding to a target sequence of the beta-catenin gene of SEQ ID NO: 173 or allele thereof and down-regulating expression of beta-catenin, said oligonucleotide comprising a sequence of 10-50 nucleobases corresponding to the target sequence.
2. The antisense oligonucleotide of embodiment 1, wherein the target sequence is selected from regions of the beta-catenin gene represented by SEQ ID NOS: 1-132 or an allelic variant thereof.
3. The oligonucleotide of embodiment 1 or embodiment 2 comprising a sequence of 10-16 nucleobases shown in SEQ ID NOS: 1, 16, 17, 18, 33, 34, 49, 50, 51, 52, 53, 54, 55, 56, 57, 58, 73, 88, 103, and 118 or an allelic variant thereof.
4. The oligonucleotide according to any one of embodiments 1-3 comprising sequences shown as SEQ ID NOS: 1-132.
5. The oligonucleotide of any one of the preceding embodiments represented by SEQ ID NOS: 133-172.
6. The oligonucleotide according to any one of the preceding embodiments, wherein said nucleobase sequence comprises internucleobase linkages independently selected from phosphodiester, phosphorothioate and boranophosphate.
7. The oligonucleotide of any one of the preceding embodiments, wherein at least two of said nucleobases are nucleotide analogues.
8. The oligonucleotide according to embodiment 5, wherein said sequence of nucleobases comprises, in a 5' to 3' direction (i) region A: a stretch of 2-4 nucleotide analogues, followed by (ii) region B: a stretch of 6-11 nucleotides or nucleotide analogues different from those of region A, followed by (iii) region C: a stretch of 2-4 nucleotide analogues, and optionally followed by (iv) region D: one or two nucleotides.
9. The oligonucleotide according to embodiment 8, wherein the region A comprises at least one phosphodiester linkage between two nucleotide analogue units and/or or a nucleotide analogue unit and a nucleobase unit of Region B.
10. The oligonucleotide according to embodiment 8 or embodiment 9,
   wherein region C comprises at least one phosphodiester linkage between two nucleotide analogue units and/or a nucleotide analogue unit and a nucleobase unit of Region B.
11. The oligonucleotide according to any one of embodiments 1 to 6,
   wherein all the internucleobase linkages are phosphorothioate.
12. The oligonucleotide according to any one of embodiments 7 to 11,
   wherein said nucleotide analogue units are independently selected from 2'-O-alkyl-RNA, 2'-amino-DNA, 2'-fluoro-DNA, locked nucleic acid (LNA), arabino nucleic acid (ANA), 2'-fluoro-ANA, HNA, INA and 2'-MOE.
13. The oligonucleotide according to embodiment 12, wherein the nucleotide analogues are independently selected from 2'-MOE-RNA, 2'-fluoro-DNA, and LNA.
14. The oligonucleotide according to embodiment 13, wherein at least one of said nucleotide analogues is a locked nucleic acid (LNA).
15. The oligonucleotide according to embodiment 14, wherein all the nucleotide analogues are LNA.
16. The oligonucleotide according to any one of embodiments 12 to 15,
   wherein LNA is selected from beta-D-oxy-LNA, alpha-L-oxy-LNA, beta-D-amino-LNA and beta-D-thio-LNA.
17. A conjugate comprising an oligonucleotide of any one of the preceding embodiments and at least one non-nucleotide or non-polynucleotide moiety covalently attached to said oligonucleotide.
18. A conjugate according to embodiment 17, wherein said non-nucleotide or non-polynucleotide moiety consists of or comprises a sterol group such as cholesterol.
19. A pharmaceutical composition comprising an oligonucleotide according to any one of the embodiments 1 to 16 or a conjugate according to embodiment 17 or embodiment 18, and a pharmaceutically acceptable diluent, carrier or adjuvant.
20. An oligonucleotide or a conjugate according to any one of embodiments 1 to 18 for use as a medicament.
21. Use of an oligonucleotide or as conjugate according to any one of embodiments 1 to 18 for the manufacture of a medicament for the treatment of abnormal levels of beta-catenin or a disease or condition for which down-regulation of beta-catenin expression is indicated.
22. The use according to embodiment 21, wherein said disease or condition is cancer.
23. A method of treating a subject suffering from cancer, the method comprising the step of administering a pharmaceutical composition, oligonucleotide or conjugate according to any one of embodiments 1 to 19 to the subject in need thereof.
24. The use or the method of any one of embodiments 21-23, wherein the cancer is selected from colorectal cancer, hepatocellular cancer, endometrial cancer, malignant melanoma, cancer of the ovary, pancreas, pituitary, oesophagus, lung, breast, kidney, haematopoetic system, cervix, CNS, bone, biliary tract and adrenal gland.
25. A target sequence within the beta-catenin gene, wherein an antisense oligonucleotide corresponding to said target sequence is capable of down-regulating the expression of beta-catenin.
26. The target sequence of embodiment 25, wherein the target sequence is selected from the regions of the beta-catenin gene complementary to SEQ ID NOs 1-132 or allelic variants thereof.
27. A method of down-regulating the expression of beta-catenin in a cell, comprising contacting the cell with an oligonucleotide according to any one of embodiments 1-16.

### EXAMPLES

### Example 1: Monomer synthesis

The LNA monomer building blocks and derivatives were prepared following published procedures and references cited therein - see WO07/031081 and the references cited therein.

### Example 2: Oligonucleotide synthesis

Oligonucleotides were synthesized according to the method described in WO07/031081. Table 1 shows examples of antisense oligonucleotide motifs and of the invention.

### Example 3: Design of the oligonucleotides

In accordance with the invention, a series of oligonucleotides were designed to target different regions of the human beta-catenin using published sequence GenBank accession number NM_001904, presented herein as SEQ ID NO: 173.

Table 2 shows oligomer designs of the invention. Table 3 shows 24mer sequence motifs from which oligomers of the invention may be designed - the bold type represents 16mer sequence motifs as shown in Table 1.

| **Table 3 - Beta-Catenin 24mers Sequences** | | | | |
|---|---|---|---|---|
| **16mer SEQ IDs** | **Shortmer SEQ IDs** | **Compoun d IDs** | **24mer SEQ IDs** | **24⁻ mer SEQ ID** |
| SEQ ID NO: 1 | 2 - 15 | 133 - 153 | ttta**gaaagctgatggacca**taac | 174 |
| SEQ ID NO: 16 | | 134 - 154 | cctc**cagacttaaagatggc**cagt | 175 |
| SEQ ID NO: 17 | | 135 - 155 | aac**acagaatccactggtga**acca | 176 |
| SEQ ID NO: 18 | 19 - 32 | 136 - 156 | aaac**gcactgccattttagc**tcct | 177 |
| SEQ ID NO: 33 | | 137 - 157 | tgtc**gtaatagccaagaatt**taac | 178 |
| SEQ ID NO: 34 | 35-48 | 138 - 158 | cagc**actctgcttgtggtcc**acag | 179 |
| SEQ ID NO: 49 | | 139 - 159 | catt**ccaccagcttctacaa**tagc | 180 |
| SEQ ID NO: 50 | | 140 - 160 | ctga**gagtccaaagacagtt**ctga | 181 |
| SEQ ID NO: 51 | | 141 - 161 | tacc**acccacttggcagacc**atca | 182 |
| SEQ ID NO: 52 | | 142 - 162 | agct**gcacaaacaatggaat**ggta | 183 |
| SEQ ID NO: 53 | | 143 - 163 | ccct**gcagctactctttgga**tgtt | 184 |
| SEQ ID NO: 54 | | 144 - 164 | gtgg**ctccctcagcttcaat**agct | 185 |
| SEQ ID NO: 55 | | 145 - 165 | atca**gcagtctcattccaag**ccat | 186 |
| SEQ ID NO: 56 | | 146 - 166 | gcca**tatccaccagagtgaa**aaga | 187 |
| SEQ ID NO: 57 | | 147 - 167 | agcc**catccatgaggtcctg**ggca | 188 |
| SEQ ID NO: 58 | 59 - 72 | 148 - 168 | **aattccatcttgtgatccattctt** | 189 |
| SEQ ID NO: 73 | 74 - 87 | 149 - 169 | ttca**aagcaagcaaagtcag**tacc | 190 |
| SEQ ID NO: 88 | 89-102 | 150 - 170 | atta**gaaattgctgtagcag**tatt | 191 |
| SEQ ID NO: 103 | 104-117 | 151 - 171 | atta**gtgttctacaccatta**ctca | 192 |
| SEQ ID NO: 118 | 119-132 | 152 - 172 | caaa**aacatgaaatagatcc**acct | 193 |

### Example 4: In vitro model: Cell culture

The effect of antisense oligonucleotides on target nucleic acid expression can be tested in any of a variety of cell types provided that the target nucleic acid is present at measurable levels. Target can be expressed endogenously or by transient or stable transfection of a nucleic acid encoding said nucleic acid. The expression level of target nucleic acid can be routinely determined using, for example, Northern blot analysis, Real-Time PCR, Ribonuclease protection assays. The following cell types are provided for illustrative purposes, but other cell types can be routinely used, provided that the target is expressed in the cell type chosen.
Cells were cultured in the appropriate medium as described below and maintained at 37°C at 95-98% humidity and 5% CO₂. Cells were routinely passaged 2-3 times weekly.

SW480: The human colorectal cancer cell line SW480 was cultured in L-15 medium (Leibovitz) + 10% fetal bovine serum (FBS) + Glutamax I + pen/strep.

HCT116: The human colorectal cancer cell line HCT116 was cultured in McCoy's 5a modified medium (Sigma) + 10% fetal bovine serum (FBS) + Glutamax I + pen/strep.

### Example 5: In vitro model: Treatment with antisense oligonucleotide

The cells were treated with oligonucleotide using the cationic liposome formulation LipofectAMINE 2000 (Gibco) as transfection vehicle. Cells were seeded in 6-well ceii culture plates (NUNC) and treated when 75-90% confluent. Oligonucleotide concentrations used ranged from 1 nM to 25 nM final concentration. Formulation of oligonucleotide-lipid complexes were carried out essentially as described by the manufacturer using serum-free OptiMEM (Gibco) and a final lipid concentration of 10 µg/mL LipofectAMINE 2000. Cells were incubated at 37°C for 4 hours and treatment was stopped by removal of oligonucleotide-containing culture medium. Cells were washed and serum-containing media was added. After oligonucleotide treatment cells were allowed to recover for 20 hours before they were harvested for RNA analysis.

### Example 6: In vitro model: Extraction of RNA and cDNA synthesis

For RNA isolation from the cell lines, the RNeasy mini kit (Qiagen cat. no. 74104) was used according to the protocol provided by the manufacturer.

First strand synthesis was performed using Reverse Transcriptase reagents from Ambion according to the manufacturer's instructions.

For each sample 0.5 µg total RNA was adjusted to (10.8 µl) with RNase free H₂O and mixed with 2 µl random decamers (50 µM) and 4 µl dNTP mix (2.5 mM each dNTP) and heated to 70 °C for 3 min after which the samples were rapidly cooled on ice. After cooling the samples on ice, 2 µl 10x Buffer RT, 1 µl MMLV Reverse Transcriptase (100 U/µl) and 0.25 µl RNase inhibitor (10 U/µl) was added to each sample, followed by incubation at 42 °C for 60 min, heat inactivation of the enzyme at 95°C for 10 min and then the sample was cooled to 4 °C.

### Example 7: In vitro model: Analysis of Oligonucleotide Inhibition of Beta-catenin Expression by Real-time PCR

Antisense modulation of beta-catenin expression can be assayed in a variety of ways known in the art. For example, beta-catenin mRNA levels can be quantitated by, e.g., Northern blot analysis, competitive polymerase chain reaction (PCR), or real-time PCR. Real-time quantitative PCR is presently preferred. RNA analysis can be performed on total cellular RNA or mRNA. Methods of RNA isolation and RNA analysis such as Northern blot analysis is routine in the art and is taught in, for example, Current Protocols in Molecular Biology, John Wiley and Sons.

Real-time quantitative (PCR) can be conveniently accomplished using the commercially available Multi-Color Real Time PCR Detection System, available from Applied Biosystem.

### Real-time Quantitative PCR Analysis of Beta-catenin mRNA Levels

The sample content of human beta-catenin mRNA was quantified using the human beta-catenin ABI Prism Pre-Developed TaqMan Assay Reagent (Applied Biosystems cat. no. Hs00170025_m1) according to the manufacturer's instructions

Glyceraldehyde-3-phosphate dehydrogenase (GAPDH) mRNA quantity was used as an endogenous control for normalizing any variance in sample preparation.

The sample content of human GAPDH mRNA was quantified using the human GAPDH ABI Prism Pre-Developed TaqMan Assay Reagent (Applied Biosystems cat. no. 4310884E) according to the manufacturer's instructions.
Real-time Quantitative PCR is a technique well known in the art and is taught in for example Heid et al. Real time quantitative PCR, Genome Research (1996), 6: 986-994.

### Real time PCR

The cDNA from the first strand synthesis performed as described in Example 8 was diluted 2-20 times, and analyzed by real time quantitative PCR using Taqman 7500 FAST from Applied Biosystems. The primers and probe were mixed with 2 x Taqman Fast Universal PCR master mix (2x) (Applied Biosystems Cat.# 4364103) and added to 4 µl cDNA to a final volume of 10 µl, Each sample was analysed in triplicate. Assaying 2 fold dilutions of a cDNA that had been prepared on material purified from a cell line expressing the RNA of interest generated standard curves for the assays. Sterile H₂O was used instead of cDNA for the no template control. PCR program: 95° C for 30 seconds, followed by 40 cycles of 95° C, 3 seconds, 60° C, 30 seconds. Relative quantities of target mRNA sequence were determined from the calculated Threshold cycle using the Applied Biosystems Fast System SDS Software Version 1.3.1.21.

### Example 8: In vitro analysis: Antisense Inhibition of Human Beta-catenin Expression by oligonucleotides

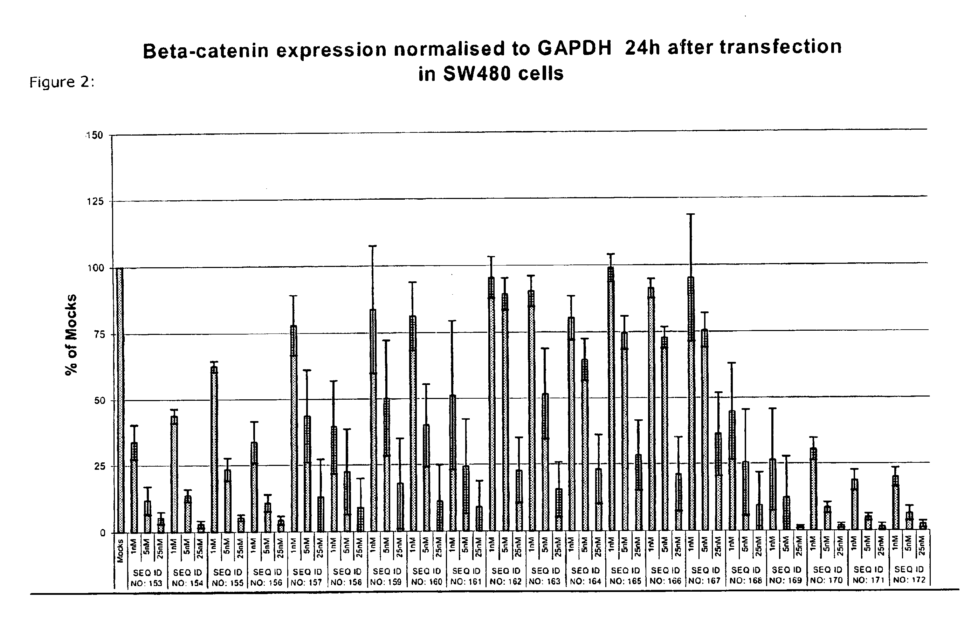

Oligonucleotides from Table 2 were evaluated for their potential to knockdown beta-catenin mRNA at concentrations of 1, 5 and 25 nM in SW480 cells (see Figure 2). The data are presented in table 4 as percentage down-regulation of beta-catenin mRNA relative to mock transfected cells at 5 nM. Lower case letters represent DNA units, bold upper case letters represent β-D-oxy-LNA units. All LNA C are 5'methyl C. Subscript "s" represents phosphorothiate linkage.

| **Table 4** | | |
|---|---|---|
| **Test substance** | **Sequence (5'-3')** | **Beta-catenin (% inhib.)** |
| SEQ ID NO: 153 | **GₛAₛAₛ**aₛgₛcₛtₛgₛaₛtₛgₛgₛaₛ**CₛCₛA** | **88.11%** |
| SEQ ID NO: 154 | **CₛAₛGₛ**aₛcₛtₛtₛaₛaₛaₛgₛaₛtₛ**GₛGₛC** | **86.4%** |
| SEQ ID NO: 155 | **CₛAₛGₛ**aₛaₛtₛcₛcₛaₛcₛtₛgₛgₛ**TₛGₛA** | **76.6%** |
| SEQ ID NO: 156 | **GₛCₛAₛ**cₛtₛgₛcₛcₛaₛtₛtₛtₛtₛ**AₛGₛC** | **89.2%** |
| SEQ ID NO: 157 | **GₛTₛAₛ**aₛtₛaₛgₛcₛcₛaₛaₛgₛaₛ**AₛTₛT** | **56.4%** |
| SEQ ID NO: 158 | **AₛCₛTₛ**cₛtₛgₛcₛtₛtₛgₛtₛgₛgₛTₛCₛC | **77.6%** |
| SEQ ID NO: 159 | **CₛCₛAₛ**CₛCₛaₛgₛcₛtₛtₛcₛtₛaₛ**cₛAₛA** | **49.7%** |
| SEQ ID NO: 160 | **GₛAₛGₛ**tₛcₛcₛaₛaₛaₛgₛaₛcₛaₛ**GₛTₛT** | **60.1%** |
| SEQ ID NO: 161 | **AₛCₛCₛ**CₛaₛCₛtₛtₛgₛgₛcₛaₛgₛ**AₛCₛC** | **75.6%** |
| SEQ ID NO: 162 | **GₛCₛAₛ**cₛaₛaₛaₛcₛaₛaₛtₛgₛgₛ**AₛAₛT** | **10.8%** |
| SEQ ID NO: 163 | **GₛCₛAₛ**gₛcₛtₛaₛcₛtₛcₛtₛtₛtₛ**GₛGₛA** | **48.3%** |
| SEQ ID NO: 164 | **CₛTₛCₛ**CₛCₛtₛCₛaₛgₛcₛtₛtₛcₛ**AₛAₛT** | **35.5%** |
| SEQ ID NO: 165 | **GₛCₛAₛ**gₛtₛcₛtₛcₛaₛtₛtₛCₛcₛ**AₛAₛG** | **25.5%** |
| SEQ ID NO: 166 | **TₛAₛTₛ**cₛcₛaₛcₛcₛaₛgₛaₛgₛtₛ**GₛAₛA** | **27.3%** |
| SEQ ID NO: 167 | **CₛAₛTₛ**cₛcₛaₛtₛgₛaₛgₛgₛtₛCₛ**CₛTₛG** | **24.5%** |
| SEQ ID NO: 168 | **CₛCₛAₛ**tₛcₛtₛtₛgₛtₛgₛaₛtₛCₛ**CₛAₛT** | **74.4%** |
| SEQ ID NO: 169 | **AₛAₛGₛ**Cₛaₛaₛgₛcₛaₛaₛaₛgₛtₛ**CₛAₛG** | **87.5%** |
| SEQ ID NO: 170 | **GₛAₛAₛ**aₛtₛtₛgₛcₛtₛgₛtₛaₛgₛ**CₛAₛG** | **91.5%** |
| SEQ ID NO: 171 | **GₛTₛGₛ**tₛtₛcₛtₛaₛcₛaₛcₛcₛaₛ**TₛTₛA** | **94.9%** |
| SEQ ID NO: 172 | **AₛAₛCₛ**aₛtₛgₛaₛaₛaₛtₛaₛgₛaₛ**TₛCₛC** | **93.6%** |
| SEQ ID NO: 194 | **CₛGₛTₛ**cₛaₛgₛtₛaₛtₛgₛcₛgₛ**AₛAₛTₛ**c | |

As shown in Table 4, oligonucleotides of SEQ ID NOs: 153, 154, 155, 156, 158, 161, 168, 169, 170, 171 and 172 demonstrated about 74% or greater inhibition of beta-catenin mRNA expression at 5 nM in these experiments and are therefore preferred.

Also preferred are oligonucleotides based on the illustrated antisense oligo sequences, for example varying the length (shorter or longer) and/or nucleobase content (e.g. the type and/or proportion of analogue units), which also provide good inhibition of beta-catenin expression.

### Example 9: In vitro analysis: Western blot analysis of Beta-catenin protein levels

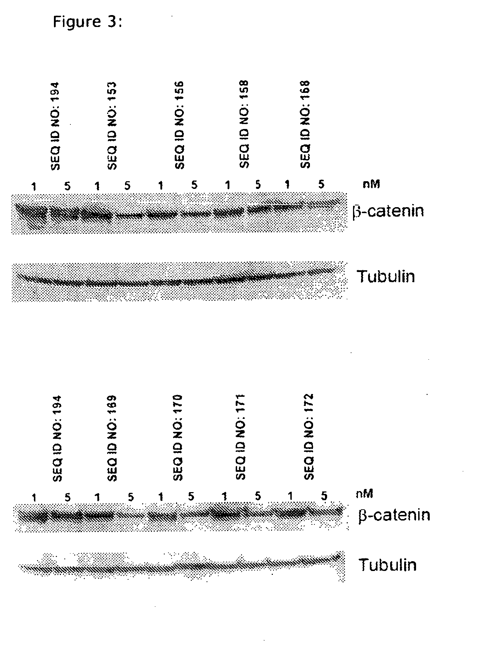

The *in vitro* effect of oligomeric compounds on beta-catenin protein levels in transfected cells was determined by Western Blotting. 200.000 SW480 cells transfected with oligonucleotides as described in Example 5 were harvested and lysed in RIPA lysis buffer (50 mM Tris-HCl pH7.4, 150mM NaCl, 1mM EDTA, 1% Triton X-100, 0.1% SDS, 1% Sodium Deoxycholate) supplemented with protease inhibitor cocktail (Roche). Total protein concentrations were measured using a BCA protein assay kit (Pierce). 50 µg total protein was run on 3-8 % Tris Acetate gels and blotted onto PVDF membranes according to manufacturer's instructions (Invitrogen). After overnight incubation in blocking buffer (PBS-T supplemented with 5% low fat milk powder), the membranes were incubated overnight with an anti-beta-catenin antibody (BD Transduction laboratories). As control of loading tubulin was detected using monoclonal antibodies from Neomarker. Membranes were then incubated with secondary antibodies and Beta-catenin or tubulin proteins were visualized using a chemiluminescens ECL⁺ detection kit (Amersham). See Figure 3.

### Example 10: Measurement of proliferating viable cells (MTS assay)

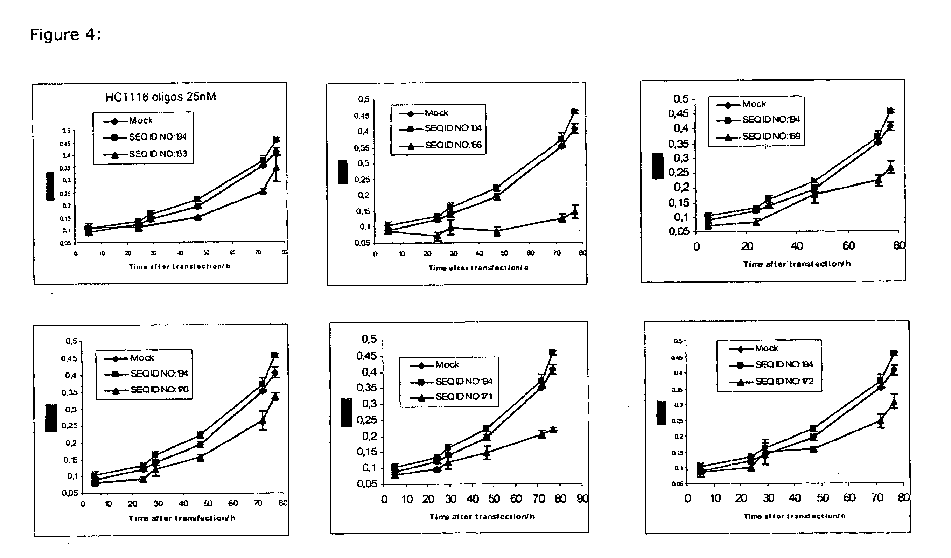

HCT116 colorectal cancer cells were seeded to a density of 200,000 cells per well in a 6 well plate in McCoy's 5a modified medium (Sigma M8403) + 2mM Glutamax I (Gibco 35050-038) + 10% FBS (Brochrom #193575010) + 25 µg/µl Gentamicin (Sigma G1397 50 mg/ml) the day prior to transfection. The next day medium was removed followed by addition of 1.2 ml OptiMEM containing 7.5 µg/ml Lipofectamine2000 (Invitrogen). Cells were incubated for 7 min before adding 0.3 ml oligonucleotides diluted in OptiMEM. The final oligonucleotide concentration was 25 nM. After 4 hours of treatment, media was removed and cells were trypsinized and seeded to a density of 5000 cells per well in clear 96 well plates (Scientific Orange no. 1472030100) in 100 µl McCoy's 5a modified medium (Sigma M8403) + 2mM Glutamax I (Gibco 35050-038) + 10% FBS (Brochrom #193575010) + 25 µg/µl Gentamicin (Sigma G1397 50 mg/ml). Viable cells were measured at the times indicated by adding 10 µl the tetrazolium compound [3-(4,5-dimethyl-2-yl)-5-(3-carboxymethoxyphenyl)-2-(4-sulfophenyl)-2H-tetrazolium, inner salt; MTS] and an electron coupling reagent (phenazine ethosulfate; PES) (CellTiter 96® AQueous One Solution Cell Proliferation Assay, Promega). Viable cells were measured at 490 nm in a Powerwave (Biotek Instruments). The OD490 nm were plotted against time/h. (See Figure 4).

### Example 11: Inhibition of beta-catenin mRNA in mouse liver

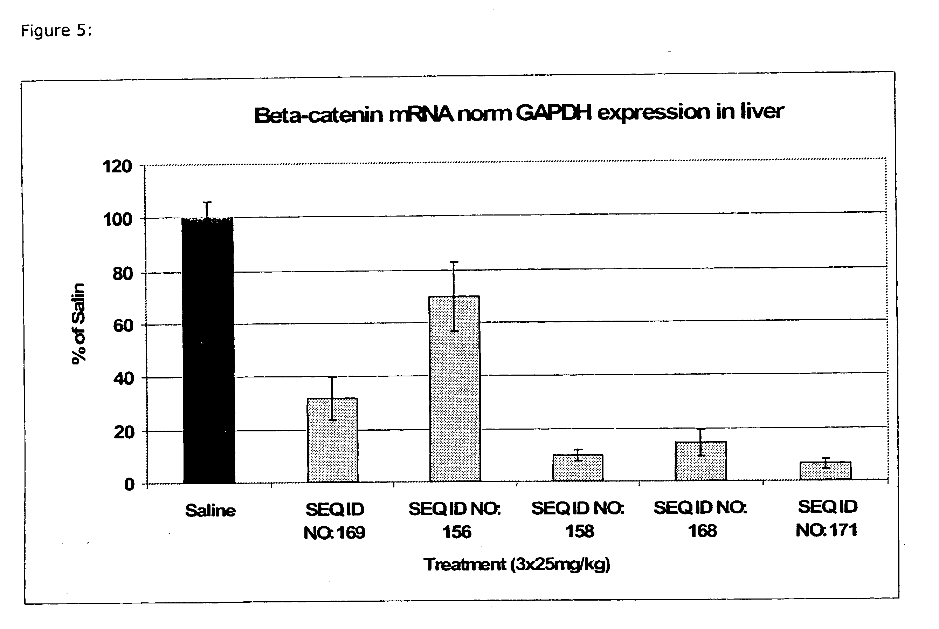

NMRI mice were dosed i.v. with 25 mg/kg oligonucleotide (SEQ ID NO: 156, 158, 168, 169, 171) on three consecutive days (group size of 5 mice). The antisense oligonucleotides were dissolved in 0.9% saline (NaCl). Animals were sacrificed 24h after last dosing and liver tissue was sampled and stored in RNA later until RNA extraction and QPCR analysis. Total RNA was extracted and beta-catenin mRNA expression in liver samples was measured by QPCR as described in Example 7 using a mouse beta-catenin QPCR assay (cat. Mm00483033_m1, Applied Biosystems). Results were normalised to mouse GAPDH (cat. no. 4352339E, Applied Biosystems) and plotted relative to saline treated controls, see Figure 5.

### Example 12: Preparation of a conjugate of SEQ ID NO: 161 and polyethylene glycol

The oligomer having SEQ ID NO: 161 is functionalized on the 5' terminus by attaching an aminoalkyl group, such as hexan-1-amine blocked with a blocking group such as Fmoc to the 5' phosphate group of the oligomer using routine phosphoramidite chemistry, oxidizing the resultant compound, deprotecting it and purifying it to achieve the functionalized oligomer having the formula (I): 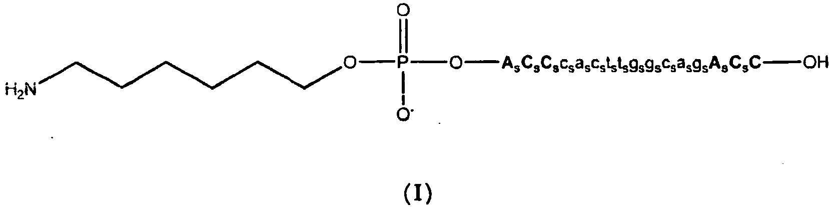 wherein the bold capital letters and subscript "s" in SEQ ID NO: 161 have the same meaning as discussed above.

A solution of activated PEG, such as the one shown in formula (II): 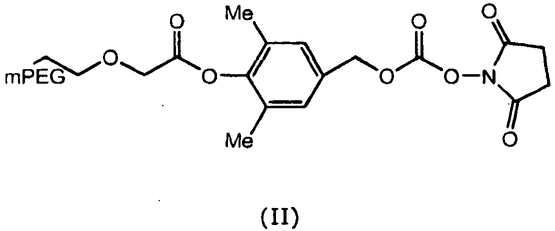 wherein the PEG moiety has an average molecular weight of 12,000, and the compound of formula (I) in PBS buffer is stirred at room temperature for 12 hours. The reaction solution is extracted three times with methylene chloride and the combined organic layers are dried over magnesium sulphate and filtered and the solvent is evaporated under reduced pressure. The residue is dissolved in double distilled water and loaded onto an anion exchange column. Unreacted PEG linker is eluted with water and the product is eluted with NH₄HCO₃ solution. Fractions containing pure product are pooled and lypophilized to yield the conjugate of formula (III): 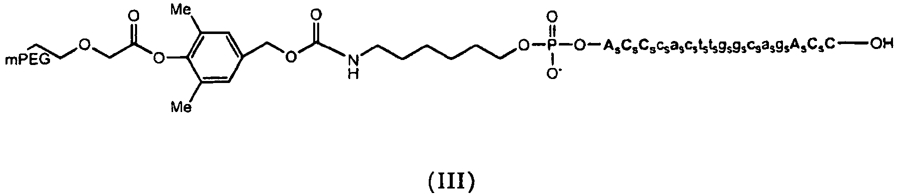 wherein the oligomer of SEQ ID NO: 161 is attached to a PEG polymer having average molecular weight of 12,000 via a releasable linker. The following are examples of conjugates of SEQ ID NO 168 and 171:

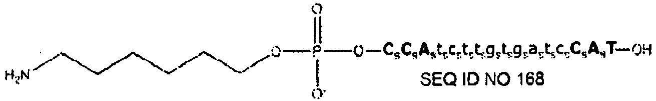

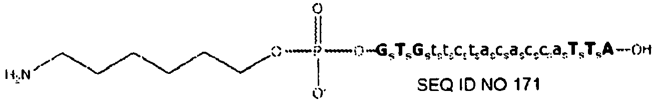

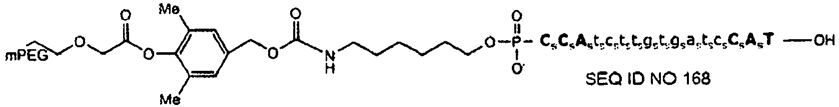

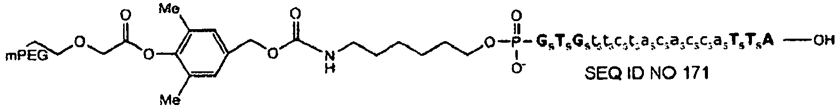

### SEQUENCE LISTING

<11C> Santaris A/S
<120> RNA ANTAGONIST COMPOUNDS FOR THE MODULATION Or BETA-CATENIN
<130> 17206PCT00
<160> 194
<170> Patent In version 3.5
<210> 1
   <211> 16
   <212> DNA
   <213> Artificial
<220>
   <223> Artificial Sequence motif
<400> 1
   gaaagtgt ggacca 16
<210> 2
   <211> 15
   <212> DNA
   <213> Artificial
<220>
   <223> Artificial Sequence motif
<400> 2
   gaaagctgat ggacc 15
<210> 3
   <211> 15
   <212> DNA
   <213> Artificial
<220>
   <223> Artificial Sequence motif
<400> 3
   aaagctgatg gacca 15
<210> 4
   <211> 14
   <212> DNA
   <213> Artificial
<220>
   <223> Artificial Sequence motif
<400> 4
   gaaagctgat ggac 14
<210> 5
   <211> 14
   <212> DNA,
   <213> Artificial
<220>
   <223> Artificial Sequence motif
<400> 5
   aaagctgatg gacc 14
<210> 6
   <211> 14
   <212> DNA
   <213> Artificial
<220>
   <223> Artificial Sequence notif
<400> 6
   aagctgatgg acca 14
<210> 7
   <211> 13
   <212> DNA
   <213> Artificial
<220>
   <223> Artificial Sequence motif
<400> 7
   gaaagctgat gga 13
<210> 8
   <211> 13
   <212> DNA
   <213> Artificial
<220>
   <223> Artificial Sequence motif
<400> 8
   aaagctgatg gac 13
<310> 9
   <211> 13
   <212> DNA
   <213> Artificial
<220>
   <223> Artificial Sequence motif
<400> 9
   aagctgatgg acc 13
<210> 10
   <211> 13
   <212> DNA
   <213> Artificial
<220>
   <223> Artificial Sequence motif
<400> 10
   agctgatgga cca 13
<210> 11
   <211> 12
   <212> LNA
   <213> Artificial
<220>
   <223> Artificial Sequence motif
<400> 11
   gaaagctgat gg 12
<210> 12
   <211> 12
   <212> DNA
   <213> Artificial
<220>
   <223> Artificial Sequence motif
<400> 12
   aaagctgatg ga 12
<210> 13
   <211> 12
   <212> DNA
   <213> Artificial
<220>
   <223> Artificial Sequence motif
<400> 13
   aagctgatgg ac 12
<210> 14
   <211> 12
   <212> DNA
   <213> Artificial
<220>
   <223> Artificial Sequence motif
<400> 14
   agctgatgga cc 12
<210> 15
   <211> 12
   <212> DNA
   <213> Artificial
<220>
   <223> Artificial Sequence motif
<400> 15
   gctgatggac ca 12
<210> 16
   <211> 16
   <212> DNA
   <213> Artificial
<220>
   <223> Artificial Sequence motif
<400> 16
   cagacttaaa gatggc 16
<210> 17
   <211> 16
   <212> DNA
   <213> Artificial
<220>
   <223> Artificial Sequence motif
<400> 17
   cagaatccac tggtga 16
<210> 18
   <211> 16
   <212> DNA
   <213> Artificial
<220>
   <223> Artificial Sequence motif
<400> 18
   gcactgccat tttagc 16
<210> 19
   <211> 15
   <212> DNA
   <213> Artificial
<220>
   <223> Artificial Sequence motif.
<400> 19
   gcactgccat tttag 15
<210> 20
   <211> 15
   <212> DNA
   <213> Artificial
<220>
   <223> Artificial Sequence motif
<400> 20
   cactgccatt ttagc 15
<210> 21
   <211> 14
   <212> DNA
   <213> Artificial
<220>
   <223> Artificial Sequence motif
<400> 21
   gcactgccat ttta 14
<210> 22
   <211> 14
   <212> DNA
   <213> Artificial
<220>
   <223> Artificial Sequence motif
<400> 22
   cactgccatt ttag 14
<210> 23
   <211> 14
   <212> DNA
   <213> Artificial
<220>
   <223> Artificial Sequence motif
<400> 23
   actgccattt tagc 14
<210> 24
   <211> 13
   <212> DNA
   <213> Artificial
<220>
   <223> Artificial Sequence motif
<400> 24
   gcactgccat ttt 13
<210> 25
   <211> 13
   <212> DNA
   <213> Artificial
<220>
   <223> Artificial Sequence motif
<400> 25
   cactgccatt tta 13
<210> 26
   <211> 13
   <212> DNA
   <213> Artificial
<220>
   <223> Artificial Sequence motif
<400> 26
   actgccattt tag 13
<210> 27
   <211> 13
   <212> DNA
   <213> Artificial
<220>
   <223> Artificial Sequence motif
<400> 27
   ctgccatttt agc 13
<210> 28
   <211> 12
   <212> DNA
   <213> Artificial
<220>
   <223> Artificial Sequence motif
<400> 28
   gcactgccat tt 12
<210> 29
   <211> 12
   <212> DNA
   <213> Artificial
<220>
   <223> Artificial Sequence motif
<400> 29
   cactgccatt tt 12
<210> 30
   <211> 12
   <212> DNA
   <213> Artificial
<220>
   <223> Artificial Sequence motif
<400> 30
   actgccattt ta 12
<210> 31
   <211> 12
   <212> DNA
   <213> Artificial
<220>
   <223> Artificial Sequence motif
<400> 31
   ctgccatttt ag 12
<210> 32
   <211> 12
   <212> DNA
   <213> Artificial
<220>
   <223> Artificial Sequence motif
<400> 32
   tgccatttta gc 12
<210> 33
   <211> 16
   <212> DNA
   <213> Artificial
<220>
   <223> Artificial Sequence motif
<400> 33
   gtaatagcca agaatt 16
<210> 34
   <211> 16
   <212> DNA
   <213> Artificial
<220>
   <223> artificial Sequence motif
<400> 34
   actctgcttg tggtcc 16
<210> 35
   <211> 15
   <212> DNA
   <213> Artificial
<220>
   <223> Artificial Sequence motif
<400> 35
   actctacttg tggtc 15
<210> 36
   <211> 15
   <212> DNA
   <213> Artificial
<220>
   <223> Artificial Sequence motif
<400> 36
   ctctgcttgt ggtcc 15
<210> 37
   <211> 14
   <212> DNA
   <213> Artificial
<220>
   <223> Artificial Sequence motif
<400> 37
   actctgcttg tggt 14
<210> 38
   <211> 14
   <212> DNA
   <213> Artificial
<220>
   <223> Artificial Sequence motif
<400> 38
   ctctgcttgt ggtc 14
<210> 39
   <211> 14
   <212> DNA
   <213> Artificial
<220>
   <223> Artificial Sequence motif
<400> 39
   tctgcttgtg gtcc 14
<210> 40
   <211> 13
   <212> DNA
   <213> Artificial
<220>
   <222> Artificial Sequence motif
<400> 40
   actctgcttg tgg 13
<210> 41
   <211> 13
   <212> DNA
   <213> Artificial
<220>
   <223> Artificial Sequence motif
<400>
   ctctgcttgtggt 13
<210> 42
   <211> 13
   <212> DNA
   <213> Artificial
<220>
   <223> Artificial Sequence motif
<400> 42
   tctgcttgtg gtc 13
<210> 43
   <211> 13
   <212> DNA
   <213> Artificial
<220>
   <223> Artificial Sequence motif
<400> 43
   ctgcttgtgg tcc 13
<210> 44
   <211> 12
   <212> DNA
   <213> Artificial
<220>
   <223> Artificial Sequence motif
<400> 44
   actctgcttg tg 12
<210> 45
   <211> 12
   <212> DNA
   <213> Artificial
<220>
   <223> Artificial Sequence motif
<400> 45
   ctctgcttgt gg 12
<210> 46
   <211> 12
   <212> DNA
   <213> Artificial
<220>
   <223> Artificial Sequence motif
<400> 46
   tctgcttgtg gt 12
<210> 47
   <211> 12
   <212> DNA
   <213> Artificial
<220>
   <223> Artificial Sequences motif
<400> 47
   ctgcttgtgg tc 12
<210> 48
   <211> 12
   <212> DNA
   <213> Artificial
<220>
   <223> Artificial Sequence motif
<400> 48
   tgcttgtggt cc 12
<210> 49
   <211> 16
   <212> DNA
   <213> Artificial
<220>
   <223> Artificial Sequence motif
<400> 49
   ccaccagctt ctacaa 16
<210> 50
   <211> 16
   <212> DNA
   <213> Artificial
<220>
   <223> Artificial Sequence motif
<400> 50
   gagtccaaag acagtt 16
<210> 51
   <211> 16
   <212> DNA
   <213> Artificial
<220>
   <223> Artificial Sequence motif
<400> 51
   acccacttgg cagacc 16
<210> 52
   <211> 16
   <212> DNA
   <213> Artificial
<220>
   <223> Artificial Sequence motif
<400> 52
   gcacaaacaa tggaat 16
<210> 53
   <211> 16
   <212> DNA
   <213> Artificial
<220>
   <223> Artificial Sequence motif
<400> 53
   gcagctactc tttgga 16
<210> 54
<-211> 16
<212> DNA
   <213> Artificial
<220>
   <223> Artificial Sequence motif
<400> 54
   ctccctcagc ttcaat 16
<210> 55
   <211> 16
   <212> DNA
   <213> Artificial
<220>
   <223> Artificial Sequence motif
<400> 55
   gcagtctcat tccaag 16
<210> 56
   <211> 16
   <212> DNA
   <213> Artificial
<220>
   <223> Artificial Sequence motif
<400> 56
   tatccaccag agtgaa 16
<210> 57
   <211> 16
   <212> DNA
   <213> Artificial
<220>
   <223> Artificial Sequence motif
<400> 57
   catccatgag gtcctg 16
<210> 58
   <211> 16
   <212> DNA
   <213> Artificial
<220>
   <223> Artificial Sequence motif
<400> 58
   ccatcttgtg atccat 16
<210> 59
   <211> 15
   <212> DNA
   <213> Artificial
<220>
   <223> Artificial Sequence motif
<400> 59
   ccatcttgtg atcca 15
<210> 60
   <211> 15
   <212> DNA
   <213> Artificial
<220>
   <223> Artificial Sequence motif
<400> 60
   catcttgtga tccat 15
<210> 61
   <211> 14
   <212> DNA
   <213> Artificial
<220>
   <223> Artificial Sequence motif
<400> 61
   ccatcttgtg atcc 14
<210> 62
   <211> 14
   <212> DNA
   <213> Artificial
<220>
   <223> Artificial Sequence motif
<400> 62
   catcttgtga tcca 14
<210> 63
   <211> 14
   <212> DNA
   <213> Artificial
<220>
   <223> Artificial Sequence motif
<400> 63
   atcttgtgat ccat 14
<210> 64
   <211> 13
   <212> DNA
   <213> Artificial
<220>
   <223> Artificial Sequence motif
<400> 64
   ccatcttgtg atc 13
<210> 65
   <211> 13
   <212> DNA
   <213> Artificial
<220>
   <223> Artificial Sequence motif
<400> 65
   catcttgtga tcc 13
<210> 66
   <211> 13
   <212> DNA
   <213> Artificial
<220>
   <223> Artificial Sequence motif
<400> 66
   atcttgtgat cca 13
<210> 67
   <211> 13
   <212> DNA
   <213> Artificial
<220>
   <223> Artificial Sequence motif
<400> 67
   tcttgtgatc cat 13
<210> 68
   <211> 12
   <212> DNA
   <213> Artificial
<220>
   <223> Artificial Sequence motif
<400> 68
   ccatcttgtg at 12
<210> 69
   <211> 12
   <212> DNA
   <213> Artificial
<220>
   <223> Artificial Sequence motif
<400> 69
   catcttgtga tc 12
<210> 70
   <211> 12
   <212> DNA
   <213> Artificial
<220>
   <220> Artificial Sequence motif
<400> 70
   atcttgtgat cc 12
<210> 71
   <211> 12
   <212> DNA
   <213> Artificial
<220>
   <223> Artificial Sequence motif
<400> 71
   tcttgtgatc ca 12
<210> 72
   <211> 12
   <212> DNA
   <213> Artificial
<220>
   <223> Artificial Sequence motif
<400> 72
   cttgtgatcc at 12
<210> 73
   <211> 16
   <212> DNA
   <213> Artificial
<220>
   <223> Artificial Sequence motif
<400> 73
   aagcaagcaa agtcag 16
<210 74
   <211> 15
   <212> DNA
   <213> Artificial
<220>
   <223> Artificial Sequence motif
<400> 74
   aagcaagcaa agtca 15
<210> 75
   <211> 15
   <212> DNA
   <213> Artificial
<220>
   <223> Artificial Sequence motif
<400> 75
   agcaagcaaa gtcag 15
<210> 76
   <211> 14
   <212> DNA
   <213> Artificial
<220>
   <223> Artificial Sequence motif
<400> 76
   aagcaagcaa agtc 14
<210> 77
   <211> 13
   <212> DNA
   <213> Artificial
<220>
   <223> Artificial Sequence motif
<400> 77
   agcaagcaaa gtc 13
<210> 78
   <211> 14
   <212> DNA
   <213> Artificial
<220>
   <223> Artificial Sequence motif
<400> 78
   gcaagcaaag tcag 14
<210> 79
   <211> 13
   <212> DNA
   <213> Artificial
<220>
   <223> Artificial Sequence motif
<400> 79
   aagcaagcaa agt 13
<210> 80
   <211> 13
   <212> DNA
   <213> Artificial
<220>
   <223> Artificial Sequence motif
<400> 80
   agcaagcaaa gtc 13
<210> 81
   <211> 13
   <212> DNA
   <213> Artificial
<220>
   <223> Artificial Sequence motif
<400> 81
   gcaaqcaaaag tca 13
<210> 82
   <211> 13
   <212> DNA
   <213> Artificial
<220>
   <223> Artificial Sequence motif
<400> 82
   caagcaaagt cag 13

<210> 83
   <211> 12
   <212> DNA
   <213> Artificial
<220>
   <223> Artiticial Sequence motif
<400> 83
   aagcaagcaa ag 12
<210> 84
   <211> 12
   <212> DNA
   <213> Artificial
<220>
   <223> Artificial Sequence motif
<400> 84
   agcaagcaaa gt 12
<210> 85
   <211> 12
   <212> DNA
   <213> Artificial
<220>
   <223> Artificial Sequence motif
<400> 85
   gcaagcaaag tc 12
<210> 86
   <211> 12
   <212> DNA
   <213> Artificial
<220>
   <223> Artificial Sequence motif
<400> 86
   caagcaaagt ca 12
<210> 87
   <211> 12
   <212> DNA
   <213> Artificial
<220>
   <223> Artificial Sequence motif
<400> 87
   aagcaaagtc ag 12
<210> 88
   <211> 16
   <212> DNA
   <213> Artificial
<220>
   <223> Artificial Sequence motif
<400> 88
   gaaattgctg tagcag 16
<210> 89
   <211> 15
   <212> DNA
   <213> Artificial
<220>
   <223> Artificial Sequence motif
<400> 89
   gaaattgctg tagca 15
<210> 90
   <211> 15
   <212> DNA
   <213> Artificial
<220>
   <223> Artificial Sequence motif
<400> 90
   aaattgctgt agcag 15
<210> 91
   <211> 14
   <212> DNA
   <213> Artificial
<220>
   <223> Artificial Sequence motif
<400> 91
   gaaattgctg tagc 14
<210> 92
   <211> 14
   <212> DNA
   <213> Artificial
<220>
   <223> Artificial Sequence motif
<400> 92
   aaattgctgt agca 14
<210> 93
   <211> 14
   <212> DNA
   <213> Artificial
<220>
   <223> Artificial Sequence motif
<400> 93
   aattgctgta gcag 14
<210> 94
   <211> 13
   <212> DNA
   <213> Artificial
<220>
   <223> Artificial Sequence motif
<400> 94
   gaaattgctg tag 13
<210> 95
   <211> 13
   <212> DNA
   <213> Artificial
<220>
   <223> Artificial Sequence motif
<400> 95
   aaattgctgt agc 13
<210> 96
   <211> 13
   <212> DNA
   <213> Artificial
<220>
   <223> Artificial Sequence motif
<400> 96
   aattgctgta gca 13
<210> 97
   <211> 13
   <212> DNA
   <213> Artificial
<220>
   <223> Artificial Sequence motif
<400> 97
   attgctgtag cag 13
<210> 98
   <211> 12
   <212> DNA
   <213> Artificial
<220>
   <223> Artificial Sequence motif
<400> 98
   gaaattgctg ta 12
<210> 99
   <211> 12
   <212> DNA
   <213> Artificial
<220>
   <223> Artificial Sequence motif
<400> 99
   aaattgctgt ag 12
<210> 100
   <211> 12
   <212> DNA
   <213> Artificial
<220>
   <223> Artificial Sequence motif
<400> 100
   adttgctgta gc 12
<210> 101
   <211> 12
   <212> DNA
   <213> Artificial
<220>
   <223> Artificial Sequence motif
<400> 101
   attgctgtag ca 12
<210> 102
   <211> 12
   <212> DNA
   <213> Artificial
<220>
   <223> Artificial Sequence motif
<400> 102
   ttactgtagc ag 12
<210> 103
   <211> 16
   <212> DNA
   <213> Artificial
<220>
   <223> Artificial Sequence notif
<400> 103
   gtgttctaca ccatta 16
<210> 104
   <211> 15
   <212> DNA
   <213> Artificial
<220>
   <223> Artificial Sequence motif
<400> 104
   gtgttctaca ccatt 15
<210> 105
   <211> 15
   <212> DNA
   <213> Artificial
<220>
   <223> Artificial Sequence motif
<400> 105
   tattctacac catta 15
<210> 106
   <211> 14
   <212> DNA
   <213> Artificial
<220>
   <223> Artificial Sequence motif
<400> 106
   gtgttctaca ccat 14
<210> 107
   <211> 14
   <212> DNA
   <213> Artificial
<220>
   <223> Artificial Sequence motif
<400> 107
   tgttctacac catt 14
<210> 108
   <211> 14
   <212> DNA
   <213> Artificial
<220>
   <223> Artificial Sequence notif
<400> 108
   gttctacacc atta 14
<210> 109
   <211> 13
   <212> DNA
   <213> artificial
<220>
   <223> Artificial Sequence motif
<400> 109
   gtgttctaca cca 13
<210> 110
   <211> 13
   <212> DNA
   <213> Artificial
<220>
   <223> Artificial Sequence motif
<400> 110
   tgttctacac cat 13
<210> 111
   <211> 13
   <212> DNA
   <213> Artificial
<220>
   <223> Artificial Sequence motif
<400> 111
   gttctacacc att 13
<210> 112
   <211> 13
   <212> DNA
   <213> Artificial
<220>
   <223> Artificial Sequence motif
<400> 112
   ttctacacca tta 13
<210> 113
   <211> 12
   <212> DNA
   <213> Artificial
<220>
   <223> Artificial Sequence motif
<400> 113
   gtgttctaca cc 12
<210> 114
   <211> 12
   <212> DNA
   <213> Artificial
<220>
   <223> Artificial Sequence motif
<40C> 114
   tgttctacac ca 12
<210> 115
   <211> 12
   <212> DNA
   <213> Artificial
<220>
   <223> Artificial Sequence notif
<400> 115
   gttctacacc at 12
<210> 116
   <211> 12
   <212> DNA
   <213> Artificial
<220>
   <223> Artificial Sequence motif
<400> 116
   ttctacacca tt 12
<210> 117
   <211> 12
   <212> DNA
   <213> Artificial

<220>
   <223> Artificial Sequence motif
<400> 117
   tctacaccat ta 12
<210> 118
   <211> 16
   <212> DNA
   <213> Artificial
<220>
   <223> Artificial Sequence motif
<400> 113
   aacatgaaat agatcc 16
<210> 119
   <211> 15
   <212> DNA
   <213> Artificial
<220>
   <223> Artificial Sequence motif
<400> 119
   aacatgaaat agatc 15
<210> 120
   <211> 15
   <212> DNA
   <213> Artificial
<220>
   <223> Artificial Sequence motifs
<400> 120
   acatgaaata gatcc 15
<210> 121
   <211> 14
   <212> DNA
   <213> Artificial
<220>
   <223> Artificial Sequence motif
<400> 121
   aacatgaaat agat 14
<210> 122
   <211> 14
   <212> DNA
   <213> Artificial
<220>
   <223> Artificial Sequence motif
<400> 122
   acatgaaata gatc 14
<210> 123
   <211> 14
   <212> DNA
   <213> Artificial
<220>
   <223> Artificial Sequence motif
<400> 123
   catgaaatag atcc 14
<210> 124
   <211> 13
   <212> DNA
   <213> Artificial
<220>
   <223> Artificial Sequence motif
<400> 124
   aacatgaaat nga 13
<210> 125
   <211> 13
   <212> DNA
   <213> Artificial
<220>
   <223> Artificial Sequence motif
<400> 125
   acatgaaata gat 13
<210> 126
   <211> 13
   <212> DNA
   <213> Artificial
<220>
   <223> Artificial Sequence motif
<400> 126
   catgaaatag atc 13
<210> 127
   <211> 13
   <212> DNA
   <213> Artificial
<220>
   <223> Artificial Sequence motif
<400> 127
   atgaaataga tcc 13
<210> 128
   <211> 12
   <212> DNA
   <213> Artificial
<220>
   <223> Artificial Sequence motif
<400> 128
   aacatgaaat ag 12
<210> 129
   <211> 12
   <212> DNA
   <213> Artificial
<220>
   <223> Artificial Sequence motif
<400> 129
   acatgaaata ga 12
<210> 130
   <211> 12
   <212> DNA
   <213> Artificial
<220>
   <223> Artificial Sequence motif
<400> 130
   catgaaatag at 12
<210> 131
   <211> 12
   <212> DNA
   <213> Artificial
<220>
   <223> Artificial Sequence motif
<400> 131
   atgaaataga tc 12
<210> 133
   <211> 12
   <212> DNA
   <213> Artificial
<220>
   <223> Artificial Sequence motif
<400> 132
   tgaaatagat cc 12
<210> 133
   <211> 16
   <212> DNA
   <213> Artificial
<220>
   <223> Oligomer designs
<220>
   <221> phosphorothiate linkage
   <222> (1)..(15)
<220>
   <221> nusleotide analogues
   <222> (1)..(3)
<220>
   <221> nucleotide analogues
   <222> (14)..(16)
<400> 133
   gaaagctgat ggacca 16
<210> 134
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 134
   cagacttaaa gatggc 16
<210> 135
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 135
   cagaatccac tggtga 16
<210> 136
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 136
   gcactgccat tttagc 16
<210> 137
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 137
   gtaatagcca agaatt 16
<210> 133
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 138
   actctgcttg tggtcc 16
<210> 139
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 139
   ccaccagctt ctacaa 16
<210> > 140
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(15)
<400> 140
   gagtccaaag acagtt 16
<210> 141
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 141
   acccacttgg cagacc 16
   <210> 142
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 142
   gcacaaacaa tggaat 16
<210> 143
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 143
   gcagctactc tttgga 16
<210> 144
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1) .. (3)
<220>
   <221> nucleotide analogues
   <222> (14)..(16)
<400> 144
   ctccctcagc ttcaat 16
<210> 145
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 145
   gcagtctcat tccaag 16
<210> 146
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(16)
<400> 146
   tatccaccag agtgaa 16
<210> 147
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (1)..(16)
<400> 147
   catccatgag gtcctg 16
<210> 148
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1).. (3)
<220>
   <221> nucleotide analogues
   <222> (14).. (16)
<400> 148
   ccatcttgtg atccat 16
<210> 149
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (:4)..(16)
<400> 199
   aagcaagcaa agtcag 16
<210> 150
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)

<220>
   <221> nucleotide analogues
   <222> (14)..(15)
<400> 150
   gaaattgctg tagcag 16
<210> 151
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotide analogues
   <222> (14)..(15)
<400> 151
   gtgttctaca ccatta 16
<210> 152
   <211> 16
   <212> DNA
   <213> Artificial
<220>
   <223> Oligomer designs
<220>
   <221> phosphorothiate linkage
   <222> (1)..(15)
<220>
   <221> nucleotide analogues
   <222> (1)..(3)
<220>
   <221> nucleotides analogues
   <222> (14)..(16)
<400> 152
   aacatgaaat agatcc 16
<210> 153
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<40C> 153
   gaaagctgat ggacca 16
<210> 154
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 154
   cagacttaaa gatggc 16
<210> 155
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 155
   cagaatccac tggtga 16
<210> 156
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 756
   gacactgccat tttagc 16
<210> 157
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 157
   gtaatagcca agaatt 16
<210> 158
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<100> 158
   actctgcttg tggtcc 16
<210> 159
   <211> 16
   <212> DNA
   <213> Artificial
<220>
   <223> Test substance
<220>
   <221> phasphorothiate linkage
   <222> (1)..(15)
<220>
   <221> LNA nucleobases
   <222> (1)..(3)
<220>
   <221> LNA nucleobases
   <222> (14)..(16)
<400> 159
   ccaccagctt ctacaa 16
<210> 160
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 160
   gagtccaaag acagtt 16
<210> 161
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 161
   acccacttgg cagacc 16
<210> 162
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 162
   gcacaaacaa tggaat 16
<210> 163
   <211> 16
   <212> DNA
   <213> Artificial
<220>
   <223> Test substance
<220>
   <221> phosphorothiate linkage
   <222> (15)
<220>
   <221> LNA nucleobases
   <222> (1)..(3)
<220>
   <221> LNA nucleobases
   <222> (14)..(16)
<400> 163
   gcagctactc tttgga 16
<210> 164
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 164
   ctccctcagc ttcaat 16
<210> 165
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1).. (3)
<220>
   <221> LNA nucleobases
   <222> (14)..(16)
<400> 165
   gcagtctcat tccaag 16
<210> 166
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 166
   tatccaccag agtgaa 16
<210> 167
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16.)
<400> 167
   catccatgag gtcctg 16
<210> 168
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1))..(3)
<220>
   <221> LNA nucleobases
   <222> (14)..(16)
<400> 168
   ccatcttgtg atccat 16
<210> 169
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 169
   aagcaagcaa agtcag 16
*<210>* 170
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 170
   gaaattgctg tagcag 16
<210> 171
   <211> 16
   <212> DNA
   <213> Artificial
<220> <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 171
   gtattctaca ccatta 16
<210> 172
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (14)..(16)
<400> 172 16
   aacatgaaat agatcc 16
<210> 173
   <211> 3697
   <212> DNA
   <213> homo sapiens
<400> 173
   cccacgcgtc cgggcagcag cgttggcccg gccccgggag cggagagcga ggggaggcgg      60
   agacggagga aggtctgagg agcagcttca gtccccgccg agccgccacc gcaggtcgag     120
   gacggtcgga ctcccgcggc gggaggagcc tgttcccctg agggtatttg aagtatacca     180
   tacaactgtt ttgaaaatcc agcgtggaca atggctactc aagctgattt gatggagttg     240
   gacatggcca tggaaccaga cagaaaagcg gctgttagtc actggcagca acagtcttac     300
   ctggactctg gaatccattc tggtgccact accacagctc cttctctgag tggtaaaggc     360
   aatcctgagg aagaggatgt ggatacctcc caagtcctgt atgagtggga acagggattt     420
   tctcagtcct tcactcaaga acaagtagct gatattgatg gacagtatgc aatgactcga     480
   gctcagaggg tacgagctgc tatgttccct gagacattag atgagggcat gcagatccca     540
   tctacacagt ttgatgctgc tcatcccact aatgtccagc gtttggctga accatcacag     600
   atgctgaaac atgcagttgt aaacttgatt aactatcaag atgatgcaga acttgccaca     660
   cgtgcaatcc ctgaactgac aaaactgcta aatgacgagg accaggtggt ggttaataag     720
   gctgcagtta tggtccatca gctttctaaa aaggaagctt ccagacacgc tatcatgcgt     780
   tctcctcaga tggtgtctgc tattgtacgt accatgcaga atacaaatga tgtagaaaca     840
   gctcgttgta ccgctgggac cttgcataac ctttcccatc atcgtgaggg cttactggcc     900
   atctttaagt ctggaggcat tcctgccctg gtgaaaatgc ttggttcacc agtggattct     960
   gtgttgtttt atgccattac aactctccac aaccttttat tacatcaaga aggagctaaa     1020
   atggcagtgc gtttagctgg tgggctgcag aaaatggttg ccttgctcaa caaaacaaat    1080
   gttaaattct tggctattac gacagactgc cttcaaattt tagcttatgg caaccaagaa    1140
   agcaagctca tcatactggc tagtggtgga ccccaagctt tagtaaatat aatgaggacc    1200
   tatacttacg aaaaactact gtggaccaca agcagagtgc tgaaggtgct atctgtctgc    1260
   tctagtaata agccggctat tgtagaagct ggtggaatgc aagctttagg acttcacctg 1320
   acagatccaa gtcaacgtct tgttcagaac tgtctttgga ctctcaggaa tctttcagat 1380
   gctgcaacta aacaggaagg gatggaaggt ctccttggga ctcttgttca gcttctgggt 1440
   tcagatgata taaatgtggt cacctgtgca gctggaattc tttctaacct cacttgcaat 1500
   aattataaga acaagatgat ggtctgccaa gtgggtggta tagaggctct tgtgcgtact 1560
   gtccttcggg ctggtgacag ggaagacatc actgagcctg ccatctgtgc tcttcgtcat 1620
   ctgaccagcc gacaccaaga agcagagatg gcccagaatg cagttcgcct tcactatgga 1680
   ctaccagttg tggttaagct cttacaccca ccatcccact ggcctctgat aaaggctact 1740
   gttggattga ttcgaaatct tgccctttgt cccgcaaatc atgcaccttt gcgtgagcag 1800
   ggtgccattc cacgactagt tcagttgctt gttcgtgcac atcaggatac ccagcgccgt 1860
   acgtccatgg gtgggacaca gcagcaattt gtggaggggg tccgcatgga agaaatagtt 1920
   gaaggttgta ccggagccct tcacatccta gctcgggatg ttcacaaccg aattgttatc 1980
   agaggactaa ataccattcc attgtttgtg cagctgcttt attctcccat tgaaaacatc 2040
   caaagagtag ctgcaggggt cctctgtgaa cttgctcagg acaaggaagc tgcagaagct 2100
   attgaagctg agggagccac agctcctctg acagagttac ttcactctag gaatgaaggt 2160
   gtggcgacat atgcagctgc tgttttgttc cgaatgtctg aggacaagcc acaagattac 2220
   aagaaacggc tttcagttga gctgaccagc tctctcttca gaacagagcc aatggcttgg 2280
   aatgagactg ctgatcttgg acttgatatt ggtgcccagg gagaacccct tggatatcgc 2340
   caggatgatc ctagctatcg ttcttttcac tctggtggat atggccagga tgccttgggt 2400
   atggacccca tgatggaaca tgagatgggt ggccaccacc ctggtgctga ctatccagtt 2460
   gatgggctgc cagatctggg gcatgcccag gacctcatgg atgggctgcc tccaggtgac 2520
   agcaatcagc tggcctggtt tgatactgac ctgtaaatca tcctttaggt aagaagtttt 2580
   aaaaagccag tttgggtaaa atacttttac tctgcctaca gaacttcaga aagacttggt 2640
   tggtagggtg ggagtggttt aggctatttg taaatctgcc acaaaaacag gtatatactt 2700
   tgaaaggaga tgtcttggaa cattggaatg ttctcagatt tctggttgtt atgtgatcat 2760
   gtgtggaagt tattaacttt aatgtttttt gccacagctt ttgcaactta atactcaaat 2820
   gagtaacatt tgctgtttta aacattaata gcagcctttc tctctttata cagctgtatt 2880
   gtctgaactt gcattgtgat tggcctgtag agttgctgag agggctcgag gggtgggctg 2940
   gtatctcaga aagtgcctga cacactaacc aagctgagtt tcctatggga acaattgaag 3000
   taaacttttt gttctggtcc tttttggtcg aggagtaaca atacaaatgg attttgggag 3060
   tgactcaaga agtgaagaat gcacaagaat ggatcacaag atggaattta gcaaacccta 3120
   gccttgcttg ttaaaatttt tttttttttt ttttaagaat atctgtaatg gtactgactt 3180
   tgcttgcttt gaagtagctc tttttttttt tttttttttt ttttttttgc agtaactgtt 3240
   ttttaagtct ctcgtagtgt taagttatag tgaatactgc tacagcaatt tctaattttt 3300
   aagaattgag taatggtgta gaacactaat taattcataa tcactctaat taattgtaat 3360
   ctgaataaag tgtaacaatt gtgtagcctt tttgtataaa atagacaaat agaaaatggt 3420
   ccaattagtt tcctttttaa tatgcttaaa ataagcaggt ggatctattt catgtttttg 3480
   atcaaaaact atttgggata tgtatgggta gggtaaatca gtaagaggtg ttatttggaa 3540
   ccttgttttg gacagtttac cagttgcctt ttatcccaaa gttgttgtaa cctgctgtga 3600
   tacgatgctt caagagaaaa tgcggttata aaaaatggtt cagaattaaa cttttaattc 3660
   attcaaaaaa aaaaaaaaaa aaaaaaaaaa aaaaaaa 3697
<210> 174
   <211> 24
   <212> DNA
   <213> Artificial
<220>
   <223> 24mer sequence motifs
<400> 174
   tttagaaagc tgatcrgacca taac 24
<210> 175
   <211> 24
   <212> DNA
   <213> Artificial
<220>
   <223> 24mer sequence motifs
<400> 175
   cctccagact taaagatgac cagt 24
<210> 176
   <211> 24
   <212> DNA
   <213> Artificial
<220>
   <223> 24mer sequence motifs
<400> 176
   aacacagaat ccactggtga acca 24
<210> 177
   <211> 24
   <212> DNA
   <213> Artificial
<220>
   <223> 24mer sequence motifs
<400> 177
   aaacgcactg ccattttagc tcct 24
<210> 178
   <211> 24
   <212> DNA
   <213> Artificial
<220>
   <223> 24mer sequence motifs
<400> 178
   tgtcgtaata gccaagaatt taac 24
<210> 179
   <211> 24
   <212> DNA
   <213> Artificial
<220>
   <223> 24mer sequence motifs
<400> 179
   cagcactctg cttgtggtcc acag 24
<210> 180
   <211> 24
   <212> DNA
   <213> Artificial
<220>
   <223> 24mer sequence motifs
<400> 180
   cattccacca gcttctacaa tagc 24
<210> 181
   <211> 24
   <212> DNA
   <213> Artificial
<220>
   <223> 24mer sequence motifs
<400> 181
   ctgagagtcc aaagacagtt ctga 24
<210> 182
   <211> 24
   <212> DNA
   <213> Artificial
<220>
   <223> 24mer sequence motifs
<400> 182
   taccacccac ttggcagacc atca 24
<210> 183
   <211> 24
   <212> DNA
   <213> Artificial
<220>
   <223> 24mer sequence motifs
<400> 183
   agctgcacaa acaatggaat ggta 24
<210> 184
   <211> 24
   <212> DNA
   <213> Artificial
<220>
   <223> 24mer sequence motifs
<400> 184
   ccctgeagct actctttgga tgtt 24
<210> 185
   <211> 24
   <212> DNA
   <213> Artificial
<220>
   <223> 24mer sequence motifs
<400> 185
   gtggctccct cagcttcaat agct 24
<210> 186
   <211> 24
   <212> DNA
   <213> Artificial
<220>
   <223> 24mer sequence motifs
<400> 186
   atcagcagtc tcattccaag ccat 24
<210> 187
   <211> 24
   <212> DNA
   <213> Artificial
<220>
   <223> 24mer sequence motifs
<400> 187
   gccatatcca ccagagtgaa aaga 24
<210> 188
   <211> 24
   <212> DNA
   <213> Artificial
<220>
   <223> 24mer sequence motifs
<400> 188
   agcccatcca tgaggtcctg ggca 24
<210> 189
   <211> 24
   <212> DNA
   <213> Artificial
<220>
   <223> 24mer sequence motifs
<400> 189
   aattccatct tgtgatccat tctt 24
<210> 190
   <211> 24
   <212> DNA
   <213> Artificial
<220>
   <223> 24mer sequence motifs
<400> 190
   ttcaagcaa gcaaagtcag tacc 24
<210> 191
   <211> 24
   <212> DNA
   <213> Artificial
<220>
   <223> 24mer sequence motifs
<400> 191
   attagaaatt gctgtagcag tatt 24
<210> 192
   <211> 24
   <212> DNA
   <213> Artificial
<220>
   <223> 24mer sequence motifs
<400> 192
   attagtgttc tacaccatta ctca 24
<210> 193
   <211> 24
   <212> DNA
   <213> Artificial
<220>
   <223> 24mer sequence motifs
<400> 193
   caaaaacatg aaatagatcc acct 24
<210> 194
   <211> 16
   <212> DNA
   <213> Artificial
<220>
   <223> Test substance
<220>
   <221> phosphorothiate linkage
   <222> (1)..(15)
<220>
   <221> LNA nucleobases
   <222> (1)..(3)
<220>
   <221> LNA nucleobases
   <222> (17.).. (15)
<400> 194 16
   catcagtatg cgaatc 16

## Claims

1. An oligomer of 10 -18 nucleobases in length, capable of inhibiting expression of the beta-catenin gene, which comprises a contiguous nucleobase sequence of a total of 10 -18 nucleobases, wherein; either i) said contiguous nucleobase sequence is 100% homologous to a corresponding region of SEQ ID NO 192; or, ii) said contiguous nucleobase sequence comprises no more than a single mismatch to a corresponding region of SEQ ID NO 192; and wherein said oligomer is a single stranded molecule.

2. The oligomer according to claim 1, wherein the contiguous nucleobase sequence comprises no mismatches with the corresponding region of SEQ ID NO 192.

3. The oligomer according to claim 1 or 2, wherein the nucleobase sequence of the oligomer consists of the contiguous nucleobase sequence.

4. The oligomer according to any one of claims 1 to 3, wherein the contiguous nucleobase sequence comprises nucleotide analogues, such as sugar modified nucleotides selected from the group consisting of: Locked Nucleic Acid (LNA) units; 2'-O-alkyl-RNA units, 2'-OMe-RNA units, 2'-amino-DNA units, and 2'-fluoro-DNA units.

5. The oligomer according to claim 4, wherein the nucleotide analogues are LNA.

6. The oligomer according to claim 4 or 5 which is a gapmer.

7. The oligomer according to any one of claims 1 to 6, which inhibits the expression of beta-catenin gene or mRNA in a cell which is expressing beta-catenin gene or mRNA.

8. The oligomer according to any one of claims 1 to 7, wherein the nucleobase sequence of said oligomer consists of SEQ ID NO 103 or SEQ ID NO 151.

9. The oligomer according to any one of claims 1 to 8, wherein the oligomer isSEQ ID NO 171: **GₛTₛGₛ**tₛtₛcₛtₛaₛcₛaₛcₛcₛaₛ**TₛTₛA**
wherein lower case letters represent DNA units; bold upper case letters represent beta-D-oxy-LNA units; all LNA C are 5'methyl C; and, subscript "s" represents phosphorothiate linkage.

10. A conjugate comprising the oligomer according to any one of claims 1 to 9, and at least one non-nucleotide or non-polynucleotide moiety covalently attached to said oligomer.

11. A pharmaceutical composition comprising the oligomer according to any one of claims 1 to 9, or the conjugate according to claim 10, and a pharmaceutically acceptable diluent, carrier, salt or adjuvant.

12. The oligomer according to any one of claims 1 to 9, or the conjugate according to claim 10, for use as a medicament, such as for the treatment of a hyperproliferative disorder, such as cancer.

13. The use of an oligomer according to any one of the claims 1 to 9, or a conjugate as defined in claim 10, for the manufacture of a medicament for the treatment of a hyperproliferative disorder, such as cancer.

14. The oligomer according to claim 12 or the use according to claim 13, wherein the oligomer is as according to claim 9.

15. An *in vitro* method for the inhibition of beta-catenin in a cell which is expressing beta-catenin, said method comprising administering an oligomer according to any one of the claims 1 to 9, or a conjugate according to claim 10 to said cell so as to inhibit beta-catenin in said cell.

## Patentansprüche

1. Oligomer mit einer Länge von 10-18 Nukleobasen, das zum Hemmen der Expression des Beta-Catenin-Gens, welches eine kontige Nukleobasensequenz von insgesamt 10-18 Nukleobasen umfasst, in der Lage ist, wobei entweder (i) die kontige Nukleobasensequenz zu einer entsprechenden Region von SEQ ID NR 192 zu 100 % homolog ist oder ii) die kontige Nukleobasensequenz nicht mehr als eine einzige Fehlpaarung mit einer entsprechenden Region von SEQ ID NR 192 umfasst; und wobei es sich bei dem Oligomer um ein einzelsträngiges Molekül handelt.

2. Oligomer nach Anspruch 1, wobei die kontige Nukleobasensequenz keine Fehlpaarungen mit der entsprechenden Region von SEQ ID Nr. 192 umfasst.

3. Oligomer nach Anspruch 1 oder 2, wobei die Nukleobasensequenz des Oligomers aus der kontigen Nukleobasensequenz besteht.

4. Oligomer nach einem der Ansprüche 1 bis 3, wobei die kontige Nukleobasensequenz Nukleotidanaloga wie etwa zuckermodifizierte Nukleotide, ausgewählt aus der Gruppe, die aus "Locked Nucleic Acid"(LNA)-Einheiten, 2'-O-Alkyl-RNA-Einheiten, 2'-OMe-RNA-Einheiten, 2'-Amino-DNA-Einheiten und 2'-Fluoro-DNA-Einheiten besteht, umfasst.

5. Oligomer nach Anspruch 4, wobei es sich bei den Nukleotidanaloga um LNA handelt.

6. Oligomer nach Anspruch 4 oder 5, bei dem es sich um ein Gapmer handelt.

7. Oligomer nach einem der Ansprüche 1 bis 8, das die Expression des Beta-Catenin-Gens oder der Beta-Catenin-mRNA in einer Zelle hemmt, welche das Beta-Catenin-Gen oder die Beta-Catenin-mRNA exprimiert.

8. Oligomer nach einem der Ansprüche 1 bis 7, wobei die Nukleobasensequenz des Oligomers aus SEQ ID NR 103 oder SEQ ID NR 151 besteht.

9. Oligomer nach einem der Ansprüche 1 bis 8, wobei es sich bei dem Oligomer um SEQ ID NR 171 handelt:
**G**ₛ**T**ₛ**G**ₛtₛtₛcₛtₛaₛcₛaₛcₛcₛaₛ**T**ₛ**T**ₛ**A**
wobei Kleinbuchstaben DNA-Einheiten wiedergeben; Großbuchstaben in Fettschrift Beta-D-Oxy-LNA-Einheiten wiedergeben; es sich bei allen LNA-C um 5'-Methyl-C handelt und das tiefgestellte "s" eine Phosphorothiat-Verknüpfung wiedergibt.

10. Konjugat, umfassend das Oligomer nach einem der Ansprüche 1 bis 9 und mindestens eine Nichtnukleotid- oder Nichtpolynukleotideinheit, die kovalent an dem Oligomer angebracht ist.

11. Pharmazeutische Zusammensetzung, umfassend das Oligomer nach einem der Ansprüche 1 bis 9 oder das Konjugat nach Anspruch 10 und ein pharmazeutisch geeignetes Verdünnungsmittel, einen pharmazeutisch geeigneten Träger, ein pharmazeutisch geeignetes Salz oder einen pharmazeutisch geeigneten Hilfsstoff.

12. Oligomer nach einem der Ansprüche 1 bis 9 oder das Konjugat nach Anspruch 10 für die Verwendung als Medikament, beispielsweise für die Behandlung einer hyperproliferativen Erkrankung wie etwa Krebs.

13. Verwendung eines Oligomers nach einem der Ansprüche 1 bis 9 oder eines Konjugats wie in Anspruch 10 definiert für die Herstellung eines Medikaments für die Behandlung einer hyperproliferativen Erkrankung wie etwa Krebs.

14. Oligomer nach Anspruch 12 oder die Verwendung nach Anspruch 13, wobei das Oligomer Anspruch 9 entspricht.

15. In-vitro-Verfahren für die Hemmung von Beta-Catenin in einer Zelle, die Beta-Catenin exprimiert, wobei das Verfahren das Verabreichen eines Oligomers nach einem der Ansprüche 1 bis 9 oder eines Konjugats nach Anspruch 10 an die Zelle zum Hemmen von Beta-Catenin in der Zelle umfasst.

## Revendications

1. Oligomère d'une longueur de 10 à 18 bases azotées, capable d'inhiber l'expression du gène de la bêta-caténine, qui comprend une séquence de bases azotées contiguës d'un total de 10 à 18 bases azotées, dans lequel : soit i) ladite séquence de bases azotées contiguës est 100% homologue à une région correspondante de SEQ ID N° 192, soit ii) ladite séquence de bases azotées contiguës comprend pas plus d'un mésappariement unique par rapport à une région correspondante de SEQ ID N° 192 ; et dans lequel ledit oligomère est une molécule simple brin.

2. Oligomère selon la revendication 1, dans lequel la séquence de bases azotées contiguës ne comprend aucun mésappariement par rapport à la région correspondante de SEQ ID N° 192.

3. Oligomère selon la revendication 1 ou la revendication 2, dans lequel la séquence de bases azotées de l'oligomère est constituée de la séquence de bases azotées contiguës.

4. Oligomère selon l'une quelconque des revendications 1 à 3, dans lequel la séquence de bases azotées contiguës comprend des analogues de nucléotides tels que des nucléotides modifiés par des sucres, choisis dans le groupe constitué par : des motifs acides nucléiques bloqués (LNA, locked nucleic acids), des motifs 2'-O-alkyl-ARN, des motifs 2'-OMe-ARN, des motifs 2'-amino-ADN et des motifs 2'-fluoro-ADN.

5. Oligomère selon la revendication 4, dans lequel les analogues de nucléotides sont des LNA.

6. Oligomère selon la revendication 4 ou la revendication 5, qui est un gapmère.

7. Oligomère selon l'une quelconque des revendications 1 à 6, qui inhibe l'expression du gène de la bêta-caténine ou d'ARNm dans une cellule qui exprime le gène de la bêta-caténine ou de l'ARNm.

8. Oligomère selon l'une quelconque des revendications 1 à 7, dans lequel la séquence de bases azotées dudit oligomère est constituée de la SEQ ID N° 103 ou de la SEQ ID N° 151.

9. Oligomère selon l'une quelconque des revendications 1 à 8, dans lequel l'oligomère est la SEQ ID N° 171 : **G**ₛ**T**ₛ**G**ₛtₛtₛcₛtₛaₛcₛaₛcₛcₛaₛ**T**ₛ**T**ₛ**A**, où les minuscules représentent des motifs d'ADN ; les majuscules en gras représentent des motifs bêta-D-oxy-LNA ; tous les LNA C sont des motifs 5'-méthyle C ; et l'indice "s" représente une liaison phosphorothioate.

10. Conjugué comprenant l'oligomère selon l'une quelconque des revendications 1 à 9 et au moins un fragment non nucléotidique ou non polynucléotidique rattaché de manière covalente audit oligomère.

11. Composition pharmaceutique comprenant l'oligomère selon l'une quelconque des revendications 1 à 9, ou le conjugué selon la revendication 10, et un diluant, un véhicule, un sel ou un adjuvant pharmaceutiquement acceptable.

12. Oligomère selon l'une quelconque des revendications 1 à 9, ou conjugué selon la revendication 10, destiné à être utilisé comme médicament, par exemple pour le traitement d'un trouble hyperprolifératif tel que le cancer.

13. Utilisation d'un oligomère selon l'une quelconque des revendications 1 à 9, ou d'un conjugué selon la revendication 10, pour fabriquer un médicament destiné au traitement d'un trouble hyperprolifératif tel que le cancer.

14. Oligomère selon la revendication 12, ou utilisation selon la revendication 13, dans lequel / laquelle l'oligomère est tel que défini dans la revendication 9.

15. Procédé *in vitro* d'inhibition de la bêta-caténine dans une cellule qui exprime de la bêta-caténine, ledit procédé comprenant l'administration à ladite cellule d'un oligomère selon l'une quelconque des revendications 1 à 9, ou d'un conjugué selon la revendication 10, afin d'inhiber la bêta-caténine dans ladite cellule.
